# EUROPEAN PATENT SPECIFICATION

(11) **EP 4 102 223 B1**
(45) Date of publication and mention of the grant of the patent: **09.12.2026**
(21) Application number: 21179093.6
(22) Date of filing: 11.06.2021
(51) Int. Cl.: G01N 33/34

(54) **METHOD FOR DETECTING A LIQUID COMPOSITION APPLIED ONTO A CELLULOSE BLANK STRUCTURE WITH A DETECTION SYSTEM AND DETECTION SYSTEM**
VERFAHREN ZUR DETEKTION EINER AUF EINE CELLULOSEROHLINGSSTRUKTUR AUFGETRAGENEN FLÜSSIGEN ZUSAMMENSETZUNG MIT EINEM DETEKTIONSSYSTEM SOWIE DETEKTIONSSYSTEM
PROCÉDÉ PERMETTANT DE DÉTECTER UNE COMPOSITION LIQUIDE APPLIQUÉE SUR UNE STRUCTURE D'ÉBAUCHE DE CELLULOSE À L'AIDE D'UN SYSTÈME DE DÉTECTION ET SYSTÈME DE DÉTECTION

(43) Date of publication of application: 14.12.2022
(73) Proprietor: PulPac AB, 421 31 Västra Frölunda (SE)
(72) Inventor: HÖGBLOM, Olle, 412 59 Göteborg (SE)
(74) Representative: Zacco Sweden AB

(56) References cited:
- WO-A1-2021/001276
- US-A- 5 338 361
- US-A1- 2009 126 889
- US-A1- 2019 120 779

## Description

### TECHNICAL FIELD

The present disclosure relates to a method for detecting a liquid composition applied onto a cellulose blank structure with a detection system. The disclosure further relates to a detection system for detecting a liquid composition applied onto a cellulose blank structure.

### BACKGROUND

Cellulose fibres are often used as raw material for producing or manufacturing cellulose products. Products formed of cellulose fibres can be used in many different situations where there is a need for having sustainable products. A wide range of cellulose products can be produced from cellulose fibres and a few examples are disposable plates and cups, cutlery, lids, bottle caps, coffee pods, and packaging materials.

One development in the field of producing cellulose products is the forming of cellulose fibres in a dry-forming process, without using traditional wet-forming methods. Instead of forming the cellulose products from a liquid or semi liquid pulp suspension or slurry, an air-formed cellulose blank structure is used. The air-formed cellulose blank structure is inserted into forming moulds of a pressing module and during the forming of the cellulose products the cellulose blank structure is subjected to a high forming pressure and a high forming temperature in the forming moulds. The pressing module may form part of a product forming unit, and the product forming unit may further comprise other systems, modules, and components, that are arranged in connection to the pressing module in the product forming unit, such as for example feeding modules, buffering modules, and blank dry forming modules.

Another system often used in the product forming unit is an application system for the application of a liquid composition onto the cellulose blank structure. Different compositions may be needed for altering the characteristics of the produced cellulose products, such as for example compositions altering mechanical, hydrophobic, and/or oleophobic properties. The cellulose blank structure is suitably a fluffy and airy structure, where the cellulose fibres forming the structures are arranged relatively loosely in relation to each other. The fluffy cellulose blank structures are used for an efficient forming of the cellulose products, allowing the cellulose fibres to form the cellulose products in an efficient way during the forming process. It is difficult to detect if the liquid composition has been applied to the cellulose blank structure in a proper way, due to the fast absorption of the composition into the fluffy fibrous structure, and because liquid compositions used commonly are transparent or white and problematic to optically distinguish from the cellulose blank structure. If the liquid composition is not applied with correct amounts and in specified areas of the cellulose blank structure, desired properties of the cellulose products may not be achieved and quality issues may arise.

There is thus a need for an improved method for detecting a liquid composition applied onto a cellulose blank structure with a detection system, and an improved detection system for detecting a liquid composition applied onto a cellulose blank structure.

US 5,338,361 A discloses apparatuses and methods for measuring and controlling the amount of multiple coating materials applied to a substrate, and for monitoring and regulating the amount of multiple coating materials containing different compositions applied to a substrate, such as paperboard.

US 2009/0126889 A1 discloses a method of monitoring and optionally controlling the addition of one or more surface additives to a papermaking process.

WO 2021/001276 A1 discloses a method for producing a cellulose product from an air-formed cellulose blank structure. In a first application step, an alkyl ketene dimer dispersion is applied to the cellulose blank structure, and applying, in a second application step, a latex dispersion to the cellulose blank structure with the applied alkyl ketene dimer dispersion.

US 2019/0120779 A1 discloses a measurement system for use in a corrugating machine and a method of checking the process for producing paper adhesions with such a measurement system

### SUMMARY

An object of the present disclosure is to provide a method for detecting a liquid composition applied onto a cellulose blank structure with a detection system, and a detection system, where the previously mentioned problems are avoided. This object is at least partly achieved by the features of the independent claims. The dependent claims contain further developments of the method for detecting a liquid composition.

The disclosure concerns a method for detecting a liquid composition applied onto a cellulose blank structure, with a detection system. The detection system is forming part of a cellulose product forming unit for forming cellulose products from the cellulose blank structure. The detection system comprises a thermal detection unit connected to a control unit. The method comprises the steps: providing the cellulose blank structure and feeding the cellulose blank structure in a feeding direction, where the cellulose blank structure upon feeding is moving through an application area and a detection area, where the application area is arranged upstream the detection area; applying the liquid composition onto the cellulose blank structure in the application area in two or more sub-steps, wherein in each sub-step a part of the liquid composition is applied onto the cellulose blank structure, wherein a temperature of each part of the liquid composition is different from the other parts to determine if a temperature deviation in the cellulose blank structure is dependent on a faulty application of either the first part or the second part of the liquid composition; detecting the applied liquid composition in the detection area with the thermal detection unit, where the detection by the thermal detection unit is forming a detection result; analysing the detection result by the control unit, where the analysis of the detection result is forming an analysis result; initiating a control action by the control unit upon detection of an analysis result deviating from a pre-determined analysis result.

Advantages with these features are that manufacturing of faulty products can be avoided through the control action. The control action is thus used for avoiding that products with defects due to improper application of the liquid composition is manufactured. Such defective products may for example have reduced ability to withstand liquids or oil based compositions. The defects are detected when the analysis result is deviating from the pre-determined analysis result. The configuration of the fluffy fibrous cellulose blank structures makes it difficult to detect if the liquid composition has been applied to the cellulose blank structure in a proper way, due to for example fast absorption of the liquid composition, and because liquid compositions used commonly are transparent or white and problematic to distinguish from the cellulose blank structure. With the method, it can efficiently be detected if the liquid composition is applied with correct amounts and in specified areas of the cellulose blank structure, due to the detection of the applied liquid composition in the detection area with the detection unit and the comparison between the analysis result and the pre-determined analysis result. The method is efficiently securing that desired properties of the cellulose products are achieved.

In one embodiment, the detection unit comprises an infrared detection sensor. The method further comprises the steps: detecting the applied liquid composition in the detection area with the infrared detection sensor, where the detection result is a temperature image of the detection area; and analysing the temperature image by the control unit for forming the analysis result. The infrared detection sensor is used for efficiently determining if the liquid composition has been correctly applied onto the cellulose blank structure through the produced temperature image. The temperature image is providing a detection result and identification of temperature value ranges or temperature value fluctuations in the temperature image may be used for determining if the liquid composition has been applied in a correct way.

According to an embodiment, the analysis result comprises temperature values of the detection area and the pre-determined analysis result comprises pre-defined temperature values. The method further comprises the step: initiating the control action by the control unit when the analysis result is a temperature value in any part of the detection area above or below the pre-defined temperature values. According to the method, the temperature values of the temperature image forming the analysis result is compared to the corresponding pre-defined temperature values of the pre-determined analysis result. If the analysis result is deviating from the pre-determined analysis result in any part, the control action is initiated, and the deviation may include threshold values that are specifying how large the deviation should be before the control action is initiated. The deviation of the temperature values of the analysis result may take place in the whole detection area, or in one or more parts of the detection area.

According to an embodiment, the analysis result comprises a mean temperature value of the detection area and the pre-determined analysis result is a pre-defined mean temperature value. The method further comprises the step: initiating the control action by the control unit when the analysis result is a mean temperature value above or below the pre-defined mean temperature value. The use of mean temperature values is one efficient way to determine the deviation in order to initiate the control action. The mean temperature value detected in the analysis result is compared to a pre-determined mean temperature value forming the pre-determined analysis result. The deviation may include a threshold value for the mean temperature value that is specifying how large the deviation should be before the control action is initiated.

In a further embodiment, the method further comprises the step: continuously detecting the applied liquid composition in the detection area by the detection unit. A continuous detection may be used for an efficient detection result through for example the use of a detected video sequence of a moving cellulose blank structure. The produced video sequence may be produced as a continuous sequence for forming the detection result in order to establish the analysis result.

In a further embodiment, the method further comprises the step: intermittently detecting the applied liquid composition in the detection area by the detection unit. An intermittent detection may be used for an efficient detection result through for example the use of a plurality of detected video sequences or still images of a moving cellulose blank structure. The produced video sequences may be produced as a plurality of following video sequences having pre-determined time intervals. Alternatively, an intermittent stream of images is produced for forming the detection result, such as a sequence of still images that is further analysed by the control unit in order to establish the analysis result.

In a further embodiment, the liquid composition is applied onto the cellulose blank structure in the application area by an application unit. The method further comprises the step: stopping the application of the liquid composition onto the cellulose blank structure by the application unit in the application area as the control action. By stopping the application of the liquid composition as the control action, it is possible to prevent that the liquid composition is applied to the cellulose blank structure in a faulty manner. When the application of the liquid composition has been stopped, further actions can be taken to ensure that the liquid composition can be applied correctly again. An operator of the cellulose product forming unit may for example be notified when the application of the liquid composition has been stopped in order to prevent that defective products are produced, by for example shutting down the cellulose product forming unit. This preventive action may also be automated by the control unit.

According to an embodiment, the method further comprises the step: initiating a cleaning operation of the application unit after stopping the application of the liquid composition onto the cellulose blank structure by the application unit. The cleaning operation is used for eliminating problems in the application unit. Such problems may for example arise if one or more of spray nozzles forming the application unit is not working properly. A malfunctioning spray nozzle may for example be clogged and the cleaning operation is securing that a clogged nozzle is working properly again.

In a further embodiment, the cellulose product forming unit comprises one or more forming moulds for forming cellulose products. The method further comprises the steps: stopping a movement of the one or more forming moulds as the control action. By stopping the forming moulds as the control action, it is possible to prevent that defective products are produced.

In further embodiments, the method further comprises the steps: marking one or more cellulose products and/or the cellulose blank structure as the control action. By marking products and/or parts of the cellulose blank structure that have not been properly applied with the liquid composition as the control action, it is possible to use a detection system for removing marked products or cellulose blank structure. The marking may for example include a colour marking operation and products or cellulose blank structure with the colour marking may be detected and removed to prevent that defective products are produced. Alternatively, the products and/or the cellulose blank structure may be marked digitally by the control unit. The control unit is storing digitally marked products or sections of the cellulose blank structure in a memory unit in order to remove the digitally marked products or sections at a later operational step where the products or parts of the cellulose blank structure for example are sorted out from the cellulose product forming unit.

In a further embodiment, the amount of liquid composition applied onto the cellulose blank structure in the application area is changed as the control action. By changing the amount of liquid composition, the deviation of the analysis result from the pre-determined analysis result can be corrected. If for example it is detected that the amount of applied liquid composition in an area of the cellulose blank structure is insufficient, the control unit could instruct the application unit to apply a higher amount of liquid composition in that specific area. Alternatively, if it is detected that the amount of applied liquid composition in an area of the cellulose blank structure is too high, the control unit could instruct the application unit to apply a lower amount of liquid composition in that specific area.

The liquid composition is applied onto the cellulose blank structure in two or more sub-steps, where in each sub-step a part of the liquid composition is applied onto the cellulose blank structure. As an example, a first row of spray nozzles may be used for spraying a first part of the liquid composition onto the cellulose blank structure and a second row of spray nozzles may be used for spraying a second part of the liquid composition onto the cellulose blank structure. The first part and the second part are together forming the liquid composition applied to the cellulose blank structure.

A temperature of each part of the liquid composition is different from the other parts. Thus in the example above, the first part and the second part of the liquid composition may have different temperatures when applied to the cellulose blank structure, and by using different temperatures the control unit could when analysing the detection result to form the analysis result determine if a temperature deviation in the cellulose blank structure is dependent on a faulty application of either the first part or the second part of the liquid composition.

The disclosure further concerns a detection system for detecting a liquid composition applied onto a cellulose blank structure upon feeding the cellulose blank structure in a feeding direction through an application area and a detection area. The detection system is forming part of a cellulose product forming unit for forming cellulose products from the cellulose blank structure. The detection system comprises a thermal detection unit connected to a control unit to determine if a temperature deviation in the cellulose blank structure is dependent on a faulty application of either a first part or a second part of the liquid composition, where the thermal detection unit is configured for detecting the applied liquid composition in the detection area arranged downstream an application area in which the liquid composition is applied onto the cellulose blank structure. The detection by the thermal detection unit is forming a detection result. The control unit is configured for analysing the detection result, and the analysis of the detection result is forming an analysis result. The control unit is configured for initiating a control action upon detection of an analysis result deviating from a pre-determined analysis result.

Advantages with these features are that manufacturing of faulty products can be avoided through the control action. The defects are detected when the analysis result is deviating from the pre-determined analysis result. The configuration of the fluffy fibrous cellulose blank structures makes it difficult to detect if the liquid composition has been applied to the cellulose blank structure in a proper way, due to for example fast absorption of the liquid composition, and because liquid compositions used commonly are transparent or white and problematic to distinguish from the cellulose blank structure. With the system, it can efficiently be detected if the liquid composition is applied with correct amounts and in specified areas of the cellulose blank structure, due to the detection of the applied liquid composition in the detection area with the detection unit and the comparison between the analysis result and the pre-determined analysis result. The system is efficiently securing that desired properties of the cellulose products are achieved.

### BRIEF DESCRIPTION OF DRAWINGS

The disclosure will be described in detail in the following, with reference to the attached drawings, in which
- Fig. 1a-b: show schematically, in a side view and in a perspective view, a cellulose product forming unit for forming cellulose products from a cellulose blank structure, where the cellulose product forming unit comprises a detection system and an application system,
- Fig. 2a-e: show schematically, in views from above, a detection area of the product forming unit, a reference temperature image, and temperature images of the detection area,
- Fig. 3a-b: show schematically, in a side view and in a perspective view, an alternative embodiment of a cellulose product forming unit, where the cellulose product forming unit comprises a detection system and an application system,
- Fig. 4a-e: show schematically, in views from above, an alternative embodiment of a detection area of the product forming unit, a reference temperature image, and temperature images of the detection area,
- Fig. 5: shows schematically, in a side view, a further alternative embodiment of a cellulose product forming unit, where the cellulose product forming unit comprises a detection system and an application system,
- Fig 6: shows schematically, a flowchart of exemplified method steps for detecting a liquid composition applied onto a cellulose blank structure.

### DESCRIPTION OF EXAMPLE EMBODIMENTS

Various aspects of the disclosure will hereinafter be described in conjunction with the appended drawings to illustrate and not to limit the disclosure, wherein like designations denote like elements, and variations of the described aspects are not restricted to the specifically shown embodiments, but are applicable on other variations of the disclosure.

Those skilled in the art will appreciate that the steps, services and functions explained herein may be implemented using individual hardware circuitry, using software functioning in conjunction with a programmed microprocessor or general purpose computer, using one or more Application Specific Integrated Circuits (ASICs) and/or using one or more Digital Signal Processors (DSPs). It will also be appreciated that when the present disclosure is described in terms of a method, it may also be embodied in one or more processors and one or more memories coupled to the one or more processors, wherein the one or more memories store one or more programs that perform the steps, services and functions disclosed herein when executed by the one or more processors.

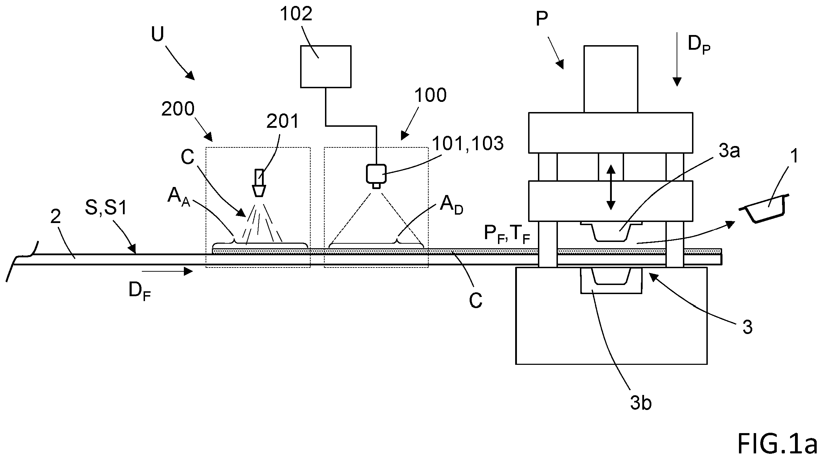

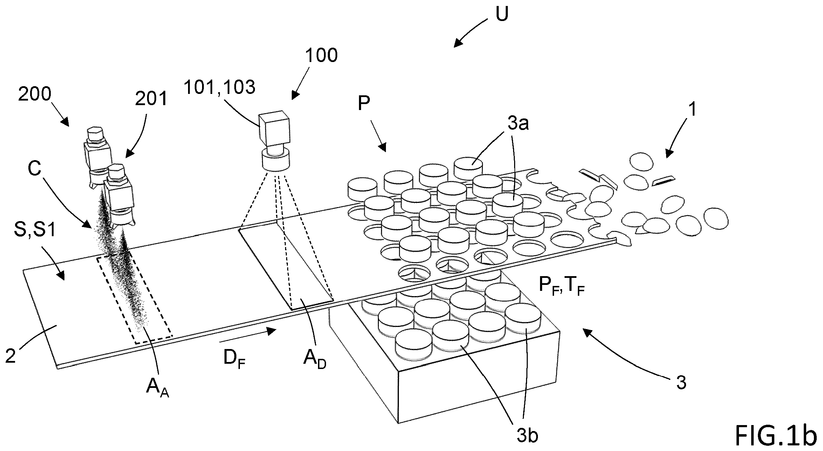

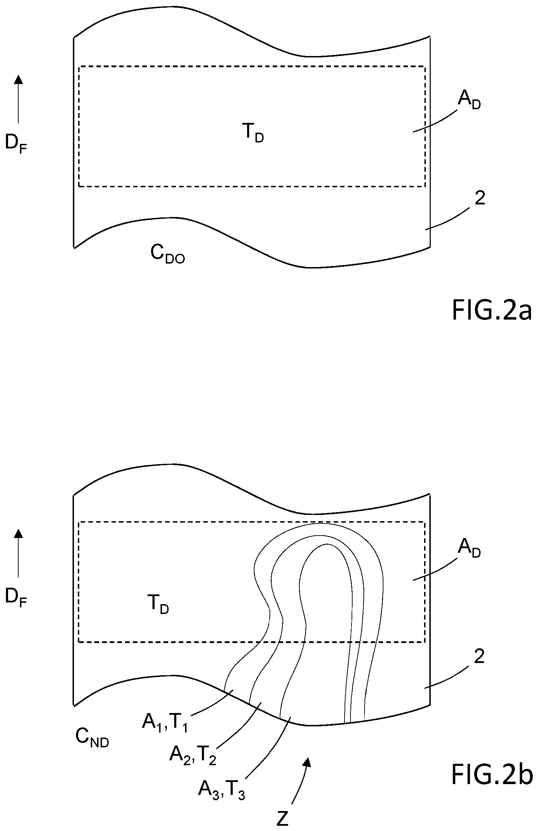

Figures 1a-b and 2a-e schematically show a first embodiment of a cellulose product forming unit U for forming cellulose products 1 from a cellulose blank structure 2 in a pressing module P.

To form the non-flat cellulose products 1 from the air-formed cellulose blank structure 2 in the product forming unit U, the cellulose blank structure 2 is first provided from a suitable source. The cellulose blank structure 2 is suitably air-formed from cellulose fibres and arranged on non-illustrated rolls or in stacks. The rolls or stacks may thereafter be arranged in connection to the product forming unit U. As an alternative, the cellulose blank structure 2 may be air-formed from cellulose fibres directly in a blank dry-forming module of the product forming unit U and fed to the pressing module P. The cellulose blank structure 2 is fed the pressing module P with suitable transportation means, such as for example forming wires, vacuum belt feeders, or conveyor belts.

With an air-formed cellulose blank structure 2 is meant an essentially air-formed fibrous web structure produced from cellulose fibres. The cellulose fibres may originate from a suitable cellulose raw material, such as a pulp material. Suitable pulp materials are for example fluff pulp, paper structures, or other cellulose fibre containing structures. With air-forming of the cellulose blank structure 2 is meant the formation of a cellulose blank structure in a dry-forming process in which the cellulose fibres are air-formed to produce the cellulose blank structure 2. When forming the cellulose blank structure 2 in the air-forming process, the cellulose fibres are carried and formed to the fibre blank structure 2 by air as carrying medium. This is different from a normal papermaking process or a traditional wet-forming process, where water is used as carrying medium for the cellulose fibres when forming the paper or fibre structure. In the air-forming process, small amounts of water or other substances may if desired be added to the cellulose fibres in order to change the properties of the cellulose product, but air is still used as carrying medium in the forming process. The cellulose blank structure 2 may, if suitable have a dryness that is mainly corresponding to the ambient humidity in the atmosphere surrounding the air-formed cellulose blank structure 2. As an alternative, the dryness of the cellulose blank structure 2 can be controlled in order to have a suitable dryness level when forming the cellulose products 1.

The air-formed cellulose blank structure 2 may be formed of cellulose fibres in a conventional air-forming process or in a blank dry-forming module. For example, the cellulose blank structure 2 may have a composition where the fibres are of the same origin or alternatively contain a mix of two or more types of cellulose fibres, depending on the desired properties of the cellulose products 1. The cellulose fibres used in the cellulose blank structure 2 are during the forming process of the cellulose products 1 strongly bonded to each other with hydrogen bonds. The cellulose fibres may be mixed with other substances or compounds to a certain amount as will be further described below. With cellulose fibres is meant any type of cellulose fibres, such as natural cellulose fibres or manufactured cellulose fibres. The cellulose blank structure 2 may specifically comprise at least 95% cellulose fibres, or more specifically at least 99% cellulose fibres.

The air-formed cellulose blank structure 2 may have a single-layer or a multi-layer configuration. A cellulose blank structure 2 having a single-layer configuration is referring to a structure that is formed of one layer containing cellulose fibres. A cellulose blank structure 2 having a multi-layer configuration is referring to a structure that is formed of two or more layers comprising cellulose fibres, where the layers may have the same or different compositions or configurations.

The cellulose blank structure 2 may comprise one or more additional cellulose layers comprising cellulose fibres, where an additional cellulose layer may be arranged as a carrying layer for one or more other layers of the cellulose blank structure 2. The one or more additional cellulose layers may act as reinforcement layers having a higher tensile strength than other layers of the cellulose blank structure 2. This is useful when one or more air-formed layers of the cellulose blank structure 2 have compositions with low tensile strength in order to avoid that the cellulose blank structure 2 will break during the forming of the cellulose products 1. The one or more additional cellulose layers with higher tensile strength act in this way as a supporting structure for other layers of the cellulose blank structure 2. The one or more additional cellulose layers may be of a different composition than the rest of the cellulose blank structure, such as for example a tissue layer containing cellulose fibres, an airlaid structure comprising cellulose fibres, or other suitable layer structures. It is thus not necessary that the one or more additional cellulose layers are air-formed. Other suitable additional layers may also be used such as for example silicone coated structures or bio-based films.

The cellulose blank structure 2 may further comprise one or more barrier layers giving the cellulose products the ability to hold or withstand liquids, such as for example when the cellulose products 1 are used in contact with beverages, food, and other water-containing substances. The barrier layer may be of a different composition than the rest of the cellulose blank structure 2, such as for example a tissue barrier structure.

The one or more air-formed layers of the cellulose blank structure 2 are fluffy and airy structures, where the cellulose fibres forming the structures are arranged relatively loosely in relation to each other. The fluffy cellulose blank structures 2 are used for an efficient forming of the cellulose products 1, allowing the cellulose fibres to form the cellulose products 1 in an efficient way during the forming process in the pressure module P.

The pressure module P comprises one or more forming moulds 3, and the one or more forming moulds 3 are configured for forming the cellulose products 1 from the cellulose blank structure 2. The pressing module P may be arranged with only one forming mould 3 in a single-cavity configuration, or alternatively with two or more forming moulds in a multi-cavity configuration. A single-cavity configuration pressing module P thus comprises only one forming mould 3 with a first mould part 3a and a cooperating second mould part 3b. A multi-cavity configuration pressing module P comprises two or more forming moulds 3, each having cooperating first mould parts 3a and second mould parts 3b.

In the embodiment illustrated in figures 1a-b, the pressing module P is arranged as a multi-cavity configuration pressing module P comprising a plurality of forming moulds 3 with first mould parts 3a and second mould parts 3b movably arranged in relation to each other, where movements of the mould parts during a product forming operation suitably are synchronized for a simultaneous pressing operation. In the following, the pressing module P will be described in connection to a multi-cavity configuration pressing module, but the disclosure is equally applicable on a single-cavity configuration pressing module.

The pressing module P can for example be constructed so that the first mould parts 3a or the second mould parts 3b are movable and arranged to move towards the other mould part during the forming process, where the other mould part is stationary or non-movably arranged. In the embodiment illustrated in figures 1a-b, the first mould parts 3a are movably arranged and the second mould parts 3b are stationary. In an alternative solution, both the first mould parts 3a and the second mould parts 3b are movably arranged, where the first mould parts 3a and the second mould parts 3b are displaced in directions towards each other during the forming process. The moving mould parts may be displaced with a suitable actuator, such as a hydraulic, pneumatic, or electric actuator. A combination of different actuators may also be used. The relative speed between the first mould part 3a and the second mould part 3b during the forming process is suitably chosen so that the cellulose blank structure 2 is evenly distributed in the one or more forming moulds 3 during the forming process.

As indicated in figure 1a, the first mould parts 3a are movably arranged in relation to the second mould parts 3b in a pressing direction D_{P} and the first mould parts 3a are further arranged to be pressed towards the second mould parts 3b during forming of the cellulose products 1 for establishing a forming pressure P_{F}. When forming the cellulose products 1, the cellulose blank structure 2 is arranged between the first mould parts 3a and the second mould parts 3b when the forming moulds 3 are in an open state. When the cellulose blank structure 2 has been arranged in the forming moulds 3, the first mould parts 3a are moved in relation to the second mould parts 3b during the forming process. When a suitable forming pressure P_{F} and forming temperature T_{F} is established in the forming moulds 3, the movement of the first mould parts 3a is stopped. The first mould parts 3a are thereafter moved in a direction away from the second mould parts 3b after a certain time period or directly after the first mould parts 3a have been stopped. A suitable control system may be used for controlling the operation of the forming moulds 3.

The cellulose products 1 are formed from the cellulose blank structure 2 in the forming moulds 3 by heating the cellulose blank structure 2 to a forming temperature T_{F} in the range of 100-300 °C, and pressing the cellulose blank structure 2 with a forming pressure P_{F} in the range of 1-100 MPa, preferably 4-20 MPa. The first mould parts 3a are arranged for forming the cellulose products 1 through interaction with corresponding second mould parts 3b, as exemplified in figures 1a-b. During forming of the cellulose products 1, the cellulose blank structure 2 is arranged in the one or more forming moulds 3, between the first mould parts 3a and the second mould parts 3b, and exerted to the forming pressure P_{F} in the range of 1-100 MPa, preferably in the range of 4-20 MPa, and the forming temperature T_{F} in the range of 100-300°C. When forming the cellulose products 1, strong hydrogen bonds are formed between the cellulose fibres in the cellulose blank structure 2 arranged between the first mould parts 3a and the second mould parts 3b. The temperature and pressure levels are for example measured in the cellulose blank structure 2 during the forming process with suitable sensors arranged in or in connection to the cellulose fibres in the cellulose blank structure 2.

The cellulose blank structure 2 is, as indicated in figures 1a-b, transported to the forming moulds 3 in a feeding direction D_{F} with a suitable transportation speed. In order to form the cellulose products 1, the cellulose blank structure 2 is arranged between the first mould parts 3a and the second mould parts 3b. When forming the cellulose products 1, a force is applied to the first mould parts 3a and/or the second mould parts 3b, and the applied force is during the forming process establishing the forming pressure P_{F} onto the cellulose blank structure 2.

The cellulose blank structure 2 may be arranged into the forming moulds 3 in any suitable way, and as an example, the cellulose blank structure 2 may be fed with a suitable feeding device, which is transporting the cellulose blank structure 2 to the forming moulds 3 in the feeding direction D_{F}. The feeding device could for example be a conveyor belt, a forming wire unit, an industrial robot, or any other suitable manufacturing equipment. The transportation speed may differ depending on the types of cellulose products 1 produced, and is chosen to match the forming speed in the forming moulds 3.

It should be understood that the one or more forming moulds 3 may have other designs and constructions compared to the ones described above, such as for example a rotary forming mould construction. The one or more forming moulds 3 may also for example be arranged with a cutting device, where the cellulose products 1 are cut into a desired shape in the one or more forming moulds 3 during the forming process. When the cellulose products 1 have been cut from the cellulose blank structure 2 in the forming process, a remaining residual cellulose fibre structure is formed. The residual cellulose fibre structure may be recycled and used again when air-forming new cellulose blank structures 2.

A desired property of the cellulose products 1 may be the ability to hold or withstand liquids, such as for example when the cellulose products are used in contact with beverages, food, and other water-containing substances. Compositions or additives used when producing cellulose products in traditional wet-forming processes are for example water based suspensions containing alkyl ketene dimer (AKD) or latex. These compositions or additives may also be used in dry-forming processes, where the air-formed cellulose blank structure 2 is used when forming the cellulose products 1 in the forming moulds 3. The compositions or additives are suitably applied to the cellulose blank structure 2 prior to the forming of the cellulose products 1 in the forming moulds 3 as a liquid composition C. The liquid compositions C may be water based and comprise any suitable additives or compositions.

The cellulose product forming unit U further comprises a detection system 100 and an application system 200 as shown in figures 1a-b. The detection system 100 is used for detecting the liquid composition C applied onto the cellulose blank structure 2. The detection system 100 comprises a detection unit 101 connected to a control unit 102. The application system 200 is applying the liquid composition C onto the cellulose blank structure 2. The application system 200 comprises an application unit 201 that is applying an amount of the liquid composition C onto a surface S of the cellulose blank structure 2. The detection system 100 and the application system 200 are forming part of the product forming unit U and may suitably be arranged as modules integrated in the product forming unit U.

As illustrated in figures 1a-b, the liquid composition C is applied to the surface S of the cellulose blank structure 2 by the application unit 201 of the application system 200. As understood from figures 1a-b, the liquid composition C is applied to the cellulose blank structure 2 in an application area A_{A}. The application area A_{A} is defining an area of the product forming unit U through which the cellulose blank structure 2 is fed in the feeding direction D_{F}. The liquid composition C is thus applied onto the cellulose blank structure 2 in the application area A_{A} upon transportation of the cellulose blank structure 2 in the feeding direction D_{F}, and the application area A_{A} may have any suitable extension in the feeding direction D_{F} and in a direction perpendicular to the feeding direction D_{F}. In the illustrated embodiment, the application area A_{A} has a rectangular-shaped configuration but it should be understood that the application area A_{A} could have any suitable shape or size. The application unit 201 may for example be arranged with a set of spray nozzles for applying the liquid composition C onto the surface S of the cellulose blank structure 2 in the application area A_{A}. In the embodiment illustrated in figures 1a-b, the liquid composition is applied from above the cellulose blank structure 2 onto a first surface S1 of the cellulose blank structure 2 with two spray nozzles forming the application unit 201. However, any suitable number of spray nozzles may be used. The spray nozzles may be of any suitable construction for distributing the liquid composition C under hydraulic or pneumatic pressure. The arrangement of spray nozzles may differ from the ones described and illustrated, depending on the configuration, shape, and size of the cellulose blank structure 2. Pulse width modulation with feedback control may suitably be used for controlling the amount of liquid composition C applied onto the cellulose blank structure 2 with the spray nozzles.

The spray nozzles of the application unit 201 in the different embodiments may spray the liquid composition C continuously or intermittently onto the cellulose blank structure 2. The liquid composition C may also be applied with the same or different amounts over the whole cellulose blank structure 2 or only on parts or zones of the cellulose blank structure 2. The spray nozzles may suitably be arranged in a non-illustrated spray booth or similar structure, preventing that the liquid composition C when sprayed are spread into the surrounding environment.

As described above, the cellulose product forming unit U further comprises the detection system 100, as shown in figures 1a-b. The detection system 100 is used for detecting that the liquid composition C is correctly applied onto the cellulose blank structure 2, and the detection system 100 comprises the detection unit 101 connected to the control unit 102.

As described above, the cellulose blank structure 2 is a fluffy and airy structure, where the cellulose fibres forming the cellulose blank structure 2 are arranged relatively loosely in relation to each other. With this configuration of the cellulose blank structure 2, it may be difficult to detect if the liquid composition C has been applied to the right area of the cellulose blank structure 2 with the right amounts of liquid composition. Therefore, the detection unit 101 is arranged in connection to a detection area A_{D} of the product forming unit U, and the detection unit 101 is used for detecting if the liquid composition C has been applied correctly.

When feeding the cellulose blank structure 2 in the feeding direction D_{F}, the cellulose blank structure 2 is moving through the application area A_{A} and the detection area A_{D}. As understood from for example figures 1a-b, the application area A_{A} is arranged upstream the detection area A_{D}.

With the method, the liquid composition C is applied onto the cellulose blank structure 2 in the application area A_{A} and thereafter the applied liquid composition C is detected in the detection area A_{D} with the detection unit 101. The detection by the detection unit 101 is forming a detection result R_{D}. The detection result R_{D} is analysed by the control unit 102, and the analysis of the detection result R_{D} is forming an analysis result R_{A}. Upon detection of an analysis result R_{A} deviating from a pre-determined analysis result R_{APD}, a control action CA is initiated by the control unit 102. The detection of the deviation of the analysis results is achieved by comparing the analysis result R_{A} with the pre-determined analysis result R_{APD}. The control action CA is preventing that cellulose products 1 with non-desired properties are produced, as will be further described below.

The detection unit 101 is a thermal imaging unit comprising infrared thermography technology. The detection unit 101 may thus comprise an infrared detection sensor 103, and the infrared detection sensor 103 is suitably a thermographic camera or similar sensor for thermal imaging or thermal video detection. The applied liquid composition C is detected in the detection area A_{D} with the infrared detection sensor 103, where the detection result R_{D} is a temperature image I of the detection area A_{D} captured by the infrared detection sensor 103. The infrared detection sensor 103 is producing temperature images I of a section of the cellulose blank structure 2 positioned in the detection area A_{D}, when the cellulose blank structure 2 is fed in the feeding direction D_{F}. The temperature image I is analysed by the control unit 102 for forming the analysis result R_{A}.

The infrared detection sensor 103 may continuously monitor the detection area A_{D}, where a continuous stream of images is produced for forming the detection result R_{D}, such as a video sequence that is further analysed by the control unit 102 in order to establish the analysis result R_{A}. The produced video sequence may be produced as a continuous sequence or alternatively as a plurality of following video sequences having pre-determined time intervals. In this embodiment, the applied liquid composition C is thus continuously detected in the detection area A_{D} by the detection unit 101.

The infrared detection sensor 103 may alternatively intermittently monitor the detection area A_{D}, where an intermittent stream of images is produced for forming the detection result R_{D}, such as video sequences or a sequence of still images that is further analysed by the control unit 102 in order to establish the analysis result R_{A}. The produced sequence may thus comprise a plurality of still images that are forming the detection result, where each image is analysed to form the analysis result R_{A}. The still images are suitably captured by the infrared detection sensor 103 with pre-determined time intervals. In this embodiment, the applied liquid composition C is thus intermittently detected in the detection area A_{D} by the detection unit 101.

The pre-determined analysis result R_{APD} can be seen as a reference temperature image I_{REF} formed as a reference still image or video sequence of the detection area A_{D} for the specific liquid composition C and cellulose blank structure 2, where the reference temperature image I_{REF} is showing a desired temperature state of the cellulose blank structure 2 with the applied liquid composition C. The reference temperature image I_{REF} may comprise one or more desired zones with pre-determined temperature ranges of the cellulose blank structure 2. If for example the whole area of the cellulose blank structure is applied with the liquid composition C, the reference temperature image I_{REF} may only comprise one temperature range zone. If for example only a part of the whole area of the cellulose blank structure is applied with the liquid composition C, the reference temperature image I_{REF} may comprise two or more temperature range zones.

In the following, the detection result R_{D} and the analysis result R_{A} will be described in connection to temperature images I captured as still images by the infrared detection sensor 103 and analysed by the control unit 102. However, the disclosure is equally applicable on a video sequence or a plurality of video sequences as the detection result R_{D}. The control unit 102 is configured for analysing still images and/or video sequences, and is arranged with software suitable for image and/or video analysis.

When the liquid composition C has been applied onto the cellulose blank structure 2, the temperature of the applied liquid composition C in the cellulose blank structure 2 is detected with the infrared detection sensor 103. If the liquid composition C is applied onto the cellulose blank structure 2 in a specific pre-determined pattern, a specific temperature pattern is established in the cellulose blank structure 2, for example through cooling effects from evaporation of the applied liquid composition C. The infrared detection sensor 103 is thus used for detecting if the liquid composition C applied onto the cellulose blank structure 2 is following the specific temperature pattern or not, and thus if the evaporation of the applied liquid composition C is occurring in a desired way. The applied liquid composition C is changing the temperature of the cellulose blank structure 2 due to for example absorption and evaporation effects of the liquid composition C when applied to and at least partly absorbed in the cellulose fibres of the cellulose blank structure 2. The control unit 102 is calibrated for a specific application of a specific liquid composition C in a specific cellulose blank structure 2, and these specific parameters are forming the pre-determined analysis result R_{APD}. The control unit 102 is programmed to determine if the produced temperature images I forming the analysis result R_{A} from the detection by the infrared detection sensor 103 are corresponding to the reference temperature image I_{REF} of the pre-determined analysis result R_{APD}, or if the produced temperature images I forming the analysis result R_{A} from the detection by the infrared detection sensor 103 are deviating from the reference temperature image I_{REF} of the pre-determined analysis result R_{APD}.

The analysis result R_{A} and the pre-determined analysis result R_{APD}, are affected by conditions such as the temperature of the applied liquid composition C and the temperature of the cellulose blank structure 2. Another condition is the amount of liquid composition C applied to the cellulose blank structure 2, and a higher amount results in a higher evaporation rate and a higher cooling effect. Other conditions affecting the results are the thickness and composition of the cellulose blank structure 2, which are impacting the absorption rate and the evaporation rate of the applied liquid composition C. The control unit 102 may therefore be programmed to consider such conditions when establishing the analysis result R_{A} and the pre-determined analysis result R_{APD}.

The control unit 102 may further be programmed with image analysis software that also considers various other conditions when determining if the produced temperature images I forming the analysis result R_{A} from the detection by the infrared detection sensor 103 are corresponding to the reference temperature image I_{REF} of the pre-determined analysis result R_{APD}. Such conditions could for example be environmental conditions, as for example temperature variations or humidity variations that are influencing the evaporation of the applied liquid composition C, and thus the analysis result R_{A}. Other conditions may be time based, where the time from spraying the liquid composition C onto the cellulose blank structure 2 to the detection by the infrared detection sensor 103 may be influencing the analysis result R_{A}. Further, minor variations or fluctuations over time may be considered, since these variations or fluctuations may indicate for example a malfunctioning spray nozzle. The control unit 102 may therefore be programmed with specific algorithms and mathematical functions that are taking these conditions into consideration when comparing the analysis result R_{A} with the pre-determined analysis result R_{APD}.

The liquid composition C applied onto the cellulose blank structure 2 in the application area A_{A} is detected with the infrared detection sensor 103 in the detection area A_{D}, and the detection result R_{D} is formed as a temperature image I of the detection area A_{D}. When analysing the detection result R_{D} in the form of the temperature image I by the control unit 102, the analysis result R_{A} is established, as will be further described below.

The analysis result R_{A} comprises temperature values T of the detection area A_{D} and the pre-determined analysis result R_{APD} comprises one or more pre-defined temperature values T_{PD} of the detection area A_{D}. It should be understood that the temperature values T and the pre-defined temperature values T_{PD} may suitably be arranged as one or more temperature ranges, and that the pre-defined temperature values T_{PD} may differ between parts or sub-areas of the reference temperature image I_{REF} forming the pre-determined analysis result R_{APD}. If the analysis result R_{A} comprises temperature values that are the same or essentially the same as the pre-defined temperature values T_{PD} of the pre-determined analysis result, it can be determined by the control unit 102 that the liquid composition C is correctly applied onto the cellulose blank structure 2. If the analysis result R_{A} comprises temperature values T that are not the same or essentially the same as the pre-defined temperature values T_{PD} of the pre-determined analysis result, it can be determined by the control unit 102 that the liquid composition C is non-correctly applied onto the cellulose blank structure 2. Thus, for a deviating analysis result R_{A}, the control unit 102 is determining that there are problems with the application of the liquid composition C onto the cellulose blank structure 2. Such problems may for example arise if one or more of the spray nozzles forming the application unit 201 is not working properly. A malfunctioning spray nozzle may for example be clogged or the supply of the liquid composition to the spray nozzle may be interrupted. In other cases, one or more of the spray nozzles may apply a too high amount of liquid composition C. The deviation of the temperature values T of the analysis result R_{A} may take place in the whole detection area A_{D}, or in one or more parts of the detection area A_{D}.

In the embodiment schematically illustrated in figures 1a-b and 2a-e, the cellulose blank structure 2 with the applied liquid composition C is fed through the detection area A_{D} in the feeding direction D_{F}. The temperature image I of the detection result R_{D} comprises detected temperatures of the cellulose blank structure in the detection area A_{D}, and the detected temperatures are when establishing the analysis result R_{A} transformed into a temperature image I comprising temperature values T of the detection area A_{D}. The temperature values T are suitably formed as one or more temperature zones, where each temperature zone has a specific temperature range. The specific temperature ranges may be based on pre-determined input values and used by the control unit 102 when forming the analysis result R_{A}. Thus, the analysis result R_{A} is in this way formed as a temperature image I comprising one or more temperature zones.

The pre-determined analysis result R_{APD} comprises one or more pre-defined temperature values T_{PD} of the detection area A_{D}, and the pre-defined temperature values T_{PD} are suitably formed as one or more temperature zones, where each temperature zone has a specific temperature range. Thus, the pre-determined analysis result R_{APD} is in this way formed as a reference temperature image I_{REF} comprising one or more temperature zones.

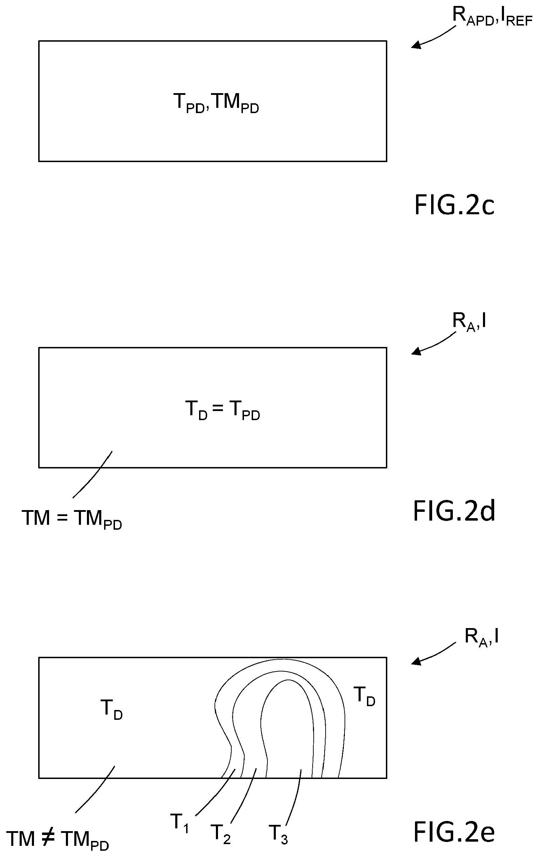

In figure 2a, the detection area A_{D} of the cellulose product forming unit U in the embodiment shown in figures 1a-b is schematically illustrated in a desired operating condition C_{DO}. In this embodiment, the liquid composition C is evenly distributed onto the cellulose blank structure 2 in the application area A_{A} in the desired operating condition C_{DO}, which is resulting in an even temperature distribution. In figure 2a, a desired temperature value T_{D} of the cellulose blank structure 2 in the desired operating condition C_{DO} is schematically illustrated, and in this embodiment it is assumed that the temperature of the cellulose blank structure 2 with the applied liquid composition C in the detection area A_{D} has the desired temperature value T_{D} or essentially the desired temperature value T_{D}. It should be understood that the desired temperature value T_{D} of the detection area A_{D} in the desired operating condition C_{DO} could include a temperature range with minor temperature variations. Based on this desired operating condition C_{DO}, the pre-determined analysis result R_{APD} can be determined, and arranged as the reference temperature image I_{REF} with the pre-defined temperature value T_{PD}. In this embodiment, the reference temperature image I_{REF} of the pre-determined analysis result R_{APD} comprises one pre-defined temperature value T_{PD}, where the pre-defined temperature value T_{PD} may be a temperature range with minor temperature variations. In figure 2c, the reference temperature image I_{REF} with the pre-defined temperature value T_{PD} of the pre-determined analysis result R_{APD} is schematically illustrated. In figure 2d, the temperature image I with the desired temperature value T_{D} of the analysis result R_{A} in the desired operating condition C_{DO} is schematically illustrated. In this case, the reference temperature image I_{REF} with the pre-defined temperature value T_{PD}, as shown in figure 2c, is corresponding to the temperature image I with the desired temperature value T_{D} in the desired operating condition C_{DO}, as shown in figure 2d.

Thus, in the desired operating conditions C_{DO}, the desired temperature value T_{D} and the pre-defined temperature value T_{PD} are the same or essentially the same, and the analysis result R_{A} in the form of the temperature image I of the detection area A_{D} based on the detection result R_{D} is corresponding to the reference temperature image I_{REF}. In the desired operating condition C_{DO}, the analysis result R_{A} is not deviating from the pre-determined analysis result R_{APD}, and no control action CA is therefore initiated.

In figure 2b, the detection area A_{D} of the cellulose product forming unit U in the embodiment shown in figures 1a-b is schematically illustrated in a non-desired operating condition C_{ND}. In the non-desired operating condition C_{ND}, the liquid composition C is unevenly distributed onto the cellulose blank structure 2 in the application area A_{A} due to for example a malfunctioning spray nozzle or interruption in the supply of the liquid composition C to a spray nozzle, which is resulting in an uneven temperature distribution. In figure 2b, a temperature zone Z of the cellulose blank structure 2 with deviating temperatures is schematically illustrated. As schematically shown in figure 2b, the temperature zone Z comprises a first area A₁ with a first deviating temperature or temperature range T₁, a second area A₂ with a second deviating temperature or temperature range T₂, and a third area A₃ with a third deviating temperature or temperature range T₃. The part of the cellulose blank structure 2 outside the temperature zone Z is having the desired temperature value T_{D} as indicated in figure 2b. Thus, in this embodiment it is assumed that the temperature of the cellulose blank structure 2 with the applied liquid composition C in the detection area A_{D} outside the temperature zone Z has the desired temperature value T_{D} or essentially the desired temperature value T_{D}, and that the temperature of the cellulose blank structure 2 with the applied liquid composition C in the temperature zone Z has temperature values deviating from the desired temperature value T_{D}. The temperature values in the temperature zone Z may thus be higher and/or lower than the desired temperature value T_{D}. In the same way as in the embodiment described above, the reference temperature image I_{REF} of the pre-determined analysis result R_{APD} comprises one pre-defined temperature value T_{PD}, where the pre-defined temperature value T_{PD} may be a temperature range with minor temperature variations. In figure 2c, the reference temperature image I_{REF} with the pre-defined temperature value T_{PD} of the pre-determined analysis result R_{APD} is schematically illustrated. In figure 2e, the temperature image I with the temperature values of the analysis result R_{A} in the non-desired operating condition C_{ND} is schematically illustrated. As schematically shown in figure 2e, a major part of the temperature image I has the desired temperature value T_{D}, and in this part it is assumed that the application of the liquid composition C is correct. Due to the deviating temperature values in the temperature zone Z, it is assumed that the application of the liquid composition C is not correct. As schematically shown in figure 2e, the temperature image I comprises a deviating first temperature or temperature range T₁, a deviating second temperature or temperature range T₂, and a deviating third temperature or temperature range T₃. The deviating temperatures or temperature ranges are higher and/or lower than the desired temperature value P_{D}. In this case, the reference temperature image I_{REF} with the pre-defined temperature value T_{PD}, as shown in figure 2c, is deviating from the temperature image I in the non-desired operating condition C_{ND}, as shown in figure 2e. Thus, in the non-desired operating conditions C_{ND}, some of the temperature values of the analysis result R_{A} and the pre-defined temperature value T_{PD} of the pre-determined analysis result R_{APD} are not the same or essentially the same, and the analysis result R_{A} in the form of the temperature image I of the detection area A_{D} based on the detection result R_{D} is not corresponding to the reference temperature image I_{REF}. In the non-desired operating condition C_{ND}, the analysis result R_{A} is deviating from the pre-determined analysis result R_{APD}, and the control action CA is therefore initiated.

The control action CA may be used for avoiding that cellulose products 1 with defects due to improper application of the liquid composition C is manufactured. The defects are detected when the analysis result R_{A} is deviating from the pre-determined analysis result R_{APD}, as described above.

As described above, the analysis result R_{A} may comprise temperature values T of the detection area A_{D} and the pre-determined analysis result R_{APD} is configured as the pre-defined temperature value T_{PD}. The control action CA may be initiated by the control unit 102 when the analysis result R_{A} is a temperature value T in any part of the detection area A_{D} above or below the pre-defined temperature value T_{PD}, such as for example in the case illustrated in figure 2e. The temperature values of the temperature image I forming the analysis result is compared to the pre-defined temperature values T_{PD} of the pre-determined analysis result. If the analysis result R_{A} is deviating from the pre-determined analysis result in any part, the control action CA is initiated. The deviation may include threshold values that are specifying how large the deviation should be before the control action is initiated. The deviation of the temperature values of the analysis result may take place in the whole detection area A_{D}, or in one or more parts of the detection area A_{D}.

Alternatively, the analysis result R_{A} may instead comprise a mean temperature value TM of the detection area A_{D} and the pre-determined analysis result R_{APD} is configured as a pre-defined mean temperature value TM_{PD}. The control action CA may be initiated by the control unit 102 when the analysis result R_{A} is a mean temperature value TM above or below the pre-defined mean temperature value TM_{PD}. The use of mean temperature values is an alternative suitable way to determine the deviation in order to initiate the control action. The mean temperature value detected in the analysis result is compared to a pre-determined mean temperature value forming the pre-determined analysis result. The deviation may include a threshold value for the mean temperature value that is specifying how large the deviation should be before the control action is initiated. In figure 2c, the pre-defined mean temperature value TM_{PD} is determined as the mean temperature value of the reference temperature image I_{REF}. The analysis result R_{A} in figure 2d is having a mean temperature value TM that is the same as the pre-defined mean temperature value TM_{PD}, and therefore no control action CA is initiated. The analysis result R_{A} in figure 2e is having a mean temperature value TM that is different from the pre-defined mean temperature value TM_{PD}, and therefore a control action CA is initiated.

It should be understood that for all embodiments, the reference temperature image I_{REF} could instead comprise two or more temperature values for different parts of the detection area A_{D}.

The control action may be arranged in different ways. In one embodiment, the control action CA includes a stopping of the application of the liquid composition C onto the cellulose blank structure 2 by the application unit 201 in the application area A_{A}. By stopping the application of the liquid composition as the control action CA, it is possible to prevent that defective products are produced. A cleaning operation O_{C} of the application unit 201 could be initiated after stopping the application of the liquid composition C onto the cellulose blank structure 2 by the application unit 201. The cleaning operation O_{C} is used for eliminating problems in the application unit 201. Such problems may for example arise if one or more of spray nozzles forming the application unit 201 is not working properly. A malfunctioning spray nozzle may for example be clogged and the cleaning operation is securing that a clogged nozzle is working properly again. In the cleaning operation, the spray nozzles may be flushed internally and/or sprayed externally with water or other suitable cleaning liquids or fluids to remove substances that are clogging the spray nozzles or disturbing the nozzle spray pattern. The cleaning operation may include high pressure flushing or spraying for an efficient removal of substances.

In one embodiment, the control action CA includes a stopping of a movement of the one or more forming moulds 3. By stopping the forming moulds as the control action, it is possible to prevent that defective products are produced.

In one embodiment, the control action CA includes a marking of one or more cellulose products 1 and/or the cellulose blank structure 2. By marking cellulose products 1 and/or parts of the cellulose blank structure 2 that have not been properly applied with the liquid composition C as the control action CA, it is possible to use a suitable detection system for removing marked cellulose products 1 or marked sections of the cellulose blank structure 2. The marking may for example be a colour marking operation and cellulose products 1 or the cellulose blank structure 2 with the colour marking may be detected and removed to prevent that defective products are produced or further handled. The colour marking operation may for example be accomplished with a suitable spray nozzle unit that is colour marking the one or more cellulose products or one or more sections of the cellulose blank structure 2 with non-properly applied liquid composition C. Alternatively, the one or more cellulose products 1 or the one or more sections of the cellulose blank structure 2 with non-properly applied liquid composition C, may be marked digitally by the control unit. The control unit is storing the digitally marked products or sections in a memory unit in order to sort out the digitally marked products or sections at a later operational step where the marked products or sections for example are removed from the cellulose product forming unit U.

In one embodiment, the amount of liquid composition C applied onto the cellulose blank structure 2 in the application area A_{A} is changed as the control action CA. By changing the amount of liquid composition, the deviation of the analysis result R_{A} from the pre-determined analysis result R_{APD} can be corrected. If it is detected that the amount of applied liquid composition C in an area of the cellulose blank structure 2 is insufficient, the control unit 102 could instruct the application unit 201 to apply a higher amount of liquid composition C in that specific area. Alternatively, if it is detected that the amount of applied liquid composition C in an area of the cellulose blank structure 2 is too high, the control unit 102 could instruct the application unit 201 to apply a lower amount of liquid composition C in that specific area. Changing an amount of the liquid composition C also includes changing a concentration or a viscosity of the liquid composition C.

In an alternative embodiment, a UV detecting sensor could be used as the detection unit 101 instead of using the infrared detection sensor described above. The UV detection sensor is used for detecting ultraviolet light. With this configuration of the detection unit, a UV reflecting pigment composition is added to the liquid composition C, and the UV detection sensor is detecting the amount of particles applied onto the cellulose blank structure. The detection result R_{D} may be transformed into an image I as the analysis result R_{A} that is processed in a similar way as the temperature image described above and compared to a reference UV image I_{REF} as the pre-determined analysis result R_{APD}.

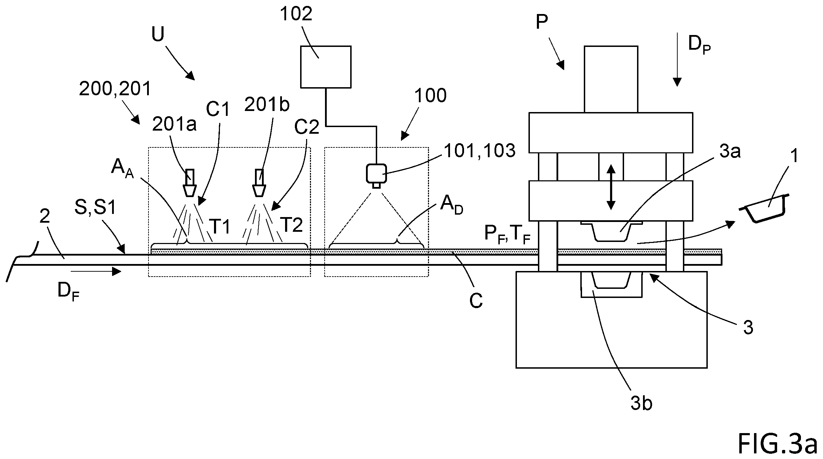

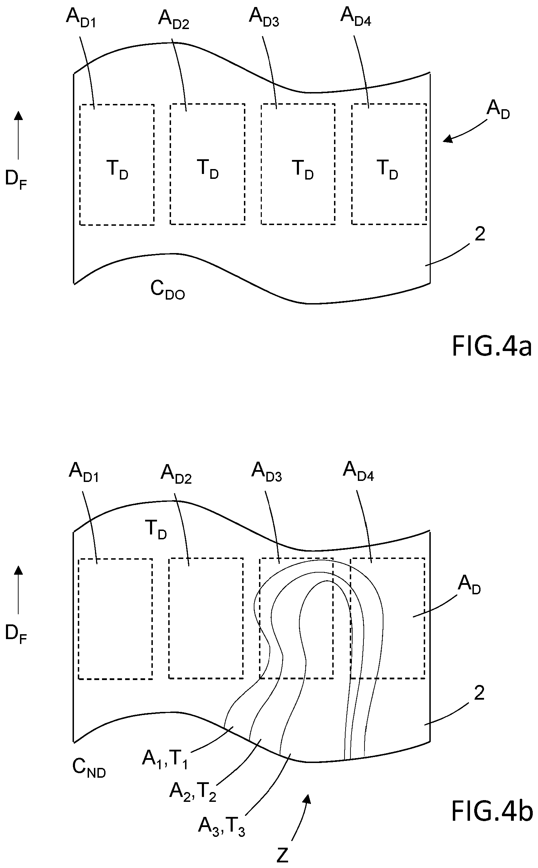

An alternative embodiment of the product forming unit U is schematically illustrated in figures 3a-b and 4a-e. In this alternative embodiment, the detection system 100 and application system 200 are having different configurations compared to the product forming unit U shown in figures 1a-b. The pressing module P may have the same configuration as the pressing module described above in connection to figures 1a-b.

As illustrated in figures 3a-b, the liquid composition C is applied to a surface S of the cellulose blank structure 2 by the application unit 201 of the application system 200. As understood from figures 3a-b, the liquid composition C is applied to the cellulose blank structure 2 in an application area A_{A}. The application area A_{A} is defining an area of the product forming unit U through which the cellulose blank structure 2 is fed in the feeding direction D_{F}. The liquid composition C is thus applied onto the cellulose blank structure 2 in the application area A_{A} upon transportation of the cellulose blank structure 2 in the feeding direction D_{F}, and the application area A_{A} may have any suitable extension in the feeding direction D_{F} and in a direction perpendicular to the feeding direction D_{F}. In the illustrated embodiment, the application area A_{A} has a rectangular-shaped configuration but it should be understood that the application area A_{A} may have any suitable shape or size. The application unit 201 may for example be arranged with a set of spray nozzles for applying the liquid composition C onto the surface S of the cellulose blank structure 2 in the application area A_{A}. In the embodiment illustrated in figures 3a-b, the liquid composition is applied from above the cellulose blank structure 2 onto a first surface S1 of the cellulose blank structure 2 with four spray nozzles arranged in two rows forming the application unit 201. However, any suitable number of spray nozzles may be used. The spray nozzles may be of any suitable construction for distributing the liquid composition C under hydraulic or pneumatic pressure. The arrangement of spray nozzles may differ from the ones described and illustrated, depending on the configuration, shape, and size of the cellulose blank structure 2.

The spray nozzles of the application unit 201 may spray the liquid composition C continuously or intermittently onto the cellulose blank structure 2. The liquid composition may also be applied over the whole cellulose blank structure 2 or only on parts or zones of the cellulose blank structure 2. The spray nozzles may suitably be arranged in a non-illustrated spray booth or similar structure, preventing that the liquid composition C when sprayed are spread into the surrounding environment.

The liquid composition C is applied onto the cellulose blank structure 2 in two or more sub-steps, and that the same or different types of compositions are applied in the different sub-steps. In the embodiment illustrated in figures 3a-b, a first row of spray nozzles 201a may be used for spraying a first part C1 of the liquid composition C onto the cellulose blank structure 2 and a second row of spray nozzles 201b may be used for spraying a second part C2 of the liquid composition C onto the cellulose blank structure 2. The first part C1 and the second part C2 are together forming the liquid composition C applied to the cellulose blank structure 2. In figures 3a-b, the first part C1 is applied upstream the second part C2, and thus the second part C2 is applied on top of the first part C1. The first part C1 and the second part C2 of the liquid composition C may have different temperatures when applied to the cellulose blank structure 2, and by using different temperatures the control unit 102 could when analysing the detection result R_{D} to form the analysis result R_{A} determine if a temperature deviation in the cellulose blank structure 2 is dependent on a faulty application of either the first part C1 or the second part C2 of the liquid composition C. Thus, the first part C1 has a first temperature T1 and the second part C2 has a second temperature T2, where the first temperature T1 is different from the second temperature T2.

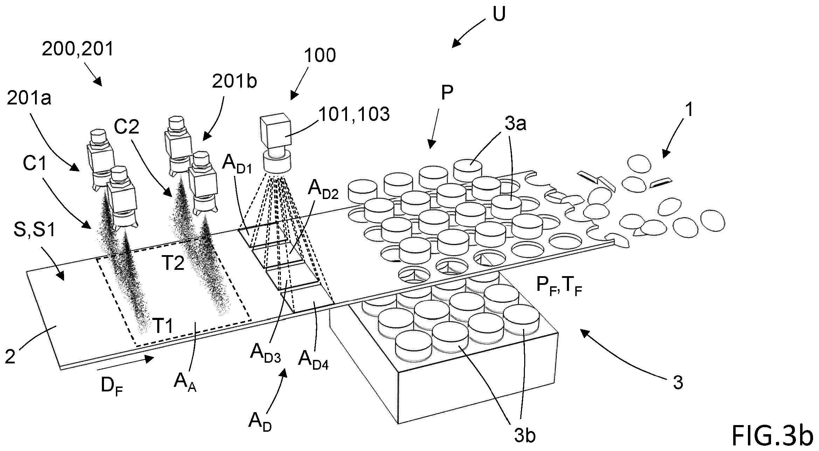

In the embodiment schematically illustrated in figures 3a-b and 4a-e, the cellulose blank structure 2 with the applied liquid composition C is fed through the detection area A_{D} in the feeding direction D_{F}. The detection area A_{D} is in this embodiment divided into four detection sub-areas arranged in connection to each other. A first detection sub-area A_{D1}, a second detection sub-area A_{D2}, a third detection sub-area A_{D3}, and a fourth detection sub-area A_{D4}, are together forming the detection area A_{D} as understood from for example figure 3b. The temperature image I of the detection result R_{D} comprises detected temperatures of the cellulose blank structure 2 in the detection sub-areas A_{D1}-A_{D4}, and the detected temperatures are when establishing the analysis result R_{A} transformed into a temperature image I in the same way as in the embodiment described above. The pre-determined analysis result R_{APD} comprises one or more pre-defined temperature values T_{PD} of the detection sub-areas A_{D1}-A_{D4}, and the pre-defined temperature values T_{PD} are suitably formed as one or more temperature zones, where each temperature zone has a specific temperature range. Thus, the pre-determined analysis result R_{APD} is in this way formed as a reference temperature image I_{REF} comprising one or more temperature zones, in the same way as described in the embodiment above in connection to figures 2a-e. It should be understood that in alternative embodiments, the detection area A_{D} could be arranged with any suitable number of sub-areas having the same or different sizes, shapes, and configurations.

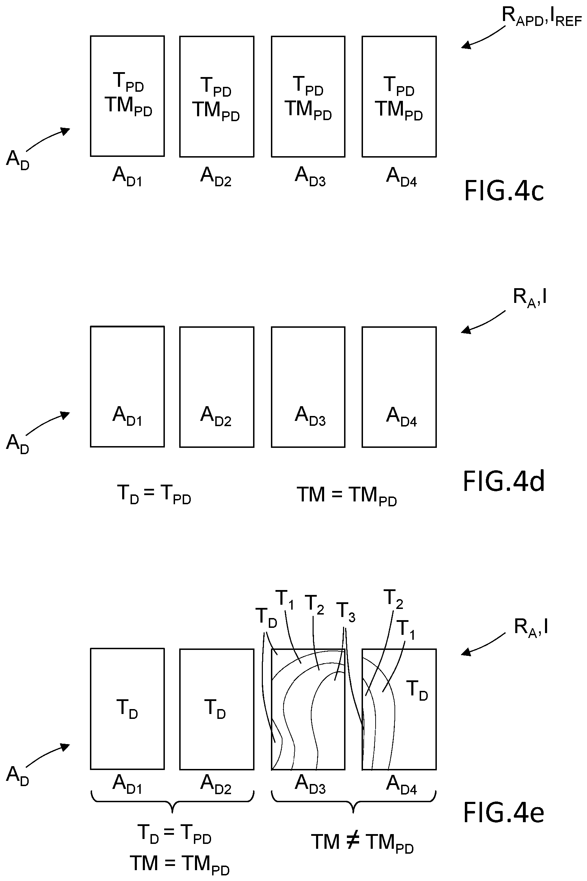

In figure 4a, the detection area A_{D} with the detection sub-areas A_{D1}-A_{D4} of the cellulose product forming unit U in the embodiment shown in figures 3a-b is schematically illustrated in a desired operating condition C_{DO}. In this embodiment, the liquid composition C is evenly distributed onto the cellulose blank structure 2 in the application area A_{A} in the desired operating condition C_{DO}, which is resulting in an even temperature distribution. In figure 4a, a desired temperature value T_{D} of the cellulose blank structure 2 in the desired operating condition C_{DO} is schematically illustrated for the detection sub-areas A_{D1}-A_{D4} and in this embodiment it is assumed that the temperature of the cellulose blank structure 2 with the applied liquid composition C in the detection area A_{D} has the desired temperature value T_{D} or essentially the desired temperature value T_{D}, in the same way as described in the embodiment above in connection to figures 2a-e. In figure 4c, the reference temperature image I_{REF} with the pre-defined temperature value T_{PD} of the pre-determined analysis result R_{APD} is schematically illustrated. In figure 4d, the temperature image I with the desired temperature value T_{D} of the analysis result R_{A} in the desired operating condition C_{DO} is schematically illustrated. In this case, the reference temperature image I_{REF} with the pre-defined temperature value T_{PD}, as shown in figure 4c, is corresponding to the temperature image I with the desired temperature value T_{D} in the desired operating condition C_{DO}, as shown in figure 4d. Thus, in the desired operating conditions C_{DO}, the desired temperature value T_{D} and the pre-defined temperature value T_{PD} are the same or essentially the same, and the analysis result R_{A} in the form of the temperature image I of the detection area A_{D} based on the detection result R_{D} is corresponding to the reference temperature image I_{REF}. In the desired operating condition C_{DO}, the analysis result R_{A} is not deviating from the pre-determined analysis result R_{APD}, and no control action CA is therefore initiated.

In figure 4b, the detection area A_{D} of the cellulose product forming unit U in the embodiment shown in figures 3a-b is schematically illustrated in a non-desired operating condition C_{ND}. In the non-desired operating condition C_{ND}, the liquid composition C is unevenly distributed onto the cellulose blank structure 2 in the application area A_{A} in the same way as described in the embodiment above, and as schematically shown in figure 4b, the temperature zone Z comprises a first area A₁ with a first deviating temperature or temperature range T₁, a second area A₂ with a second deviating temperature or temperature range T₂, and a third area A₃ with a third deviating temperature or temperature range T₃, and where the part of the cellulose blank structure 2 outside the temperature zone Z is having the desired temperature value T_{D} as indicated in figure 4b.

In figure 4e, the temperature image I with the temperature values of the analysis result R_{A} in the non-desired operating condition C_{ND} is schematically illustrated. As schematically shown in figure 4e, parts of the temperature image I, corresponding to the first detection sub-area A_{D1} and the second detection sub-area A_{D2}, have the desired temperature value T_{D}. As schematically shown in figure 4e, the third detection sub-area A_{D3} and the fourth detection sub-area A_{D4} of the temperature image I each comprises a deviating first temperature or temperature range T₁, a deviating second temperature or temperature range T₂, and a deviating third temperature or temperature range T₃. The deviating temperatures or temperature ranges are higher and/or lower than the desired temperature value P_{D}. In this case, the reference temperature image I_{REF} with the pre-defined temperature value T_{PD}, as shown in figure 4c, is deviating from the temperature image I in the non-desired operating condition C_{ND}, as shown in figure 4e. Thus, in the non-desired operating conditions C_{ND}, some of the temperature values of the analysis result R_{A} and the pre-defined temperature value T_{PD} of the pre-determined analysis result R_{APD} are not the same or essentially the same, and the analysis result R_{A} in the form of the temperature image I of the detection area A_{D} based on the detection result R_{D} is not corresponding to the reference temperature image I_{REF}. In the non-desired operating condition C_{ND}, the analysis result R_{A} is deviating from the pre-determined analysis result R_{APD}, and the control action CA is therefore initiated.

In a similar way as described in the embodiment above in connection to figures 2a-e, the control action CA may be initiated by the control unit 102 when the analysis result R_{A} is a temperature value T in any part of a detection sub-area A_{D1}-A_{D4} different from the pre-defined temperature value T_{PD}, or alternatively, when the analysis result R_{A} is a mean temperature value TM of a detection sub-area A_{D1}-A_{D4} different from the pre-defined mean temperature value TM_{PD}.

It should be understood that for all embodiments, the reference temperature image I_{REF} could instead comprise two or more temperature values for different parts of the detection area A_{D}. The control action may be arranged in the different ways described above. With the configuration of the detection area A_{D} comprising detection sub-areas, it is possible to only stop one or more forming moulds 3 corresponding to the detection sub-area as the control action CA, where forming moulds 3 corresponding to one or more detection sub-areas with deviating temperature values are stopped.

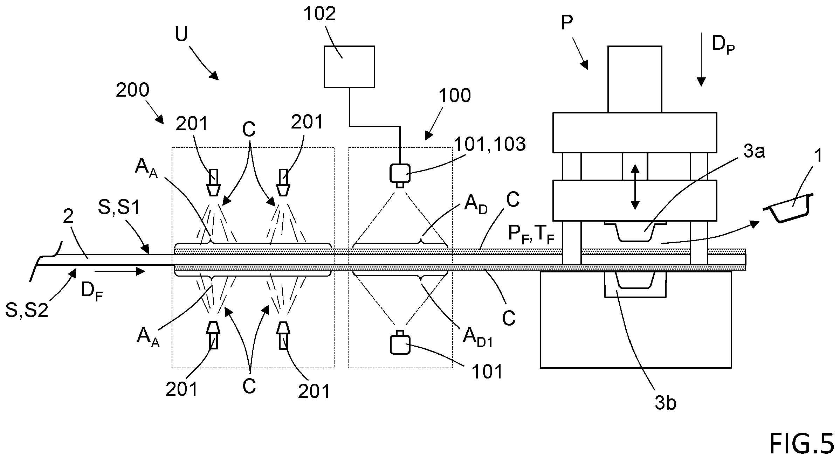

A further alternative embodiment of the product forming unit U is schematically illustrated in figure 5. In this alternative embodiment, the detection system 100 and application system 200 are having different configurations compared to the product forming unit U shown in figures 1a-b and 3a-b. The pressing module P may have the same configuration as the pressing module described above in connection to figures 1a-b.

As illustrated in figure 5, the liquid composition C is applied to surfaces S of the cellulose blank structure 2 by the application unit 201 of the application system 200. The application unit 201 may for example be arranged with an upper and a lower set of spray nozzles for applying the liquid composition C onto the surfaces S of the cellulose blank structure 2. In the embodiment illustrated in figure 5, the liquid composition is applied from above the cellulose blank structure 2 onto a first surface S1 of the cellulose blank structure 2 in an upper application area A_{A} with two spray nozzles, and onto a second surface S2 in a lower application area A_{A} with two spray nozzles, where the four spray nozzles together are forming the application unit 201. As understood from figure 5, the liquid composition C is applied to the cellulose blank structure 2 in the application areas A_{A}. The application areas A_{A} are in this embodiment defining an upper area and a lower area of the product forming unit U through which the cellulose blank structure 2 is fed in the feeding direction D_{F}. The liquid composition C is thus applied onto the cellulose blank structure 2 in the application areas A_{A} upon transportation of the cellulose blank structure 2 in the feeding direction D_{F}, and the application areas A_{A} may have any suitable extension in the feeding direction D_{F} and in a direction perpendicular to the feeding direction D_{F}. The application areas A_{A} may have any suitable shapes or sizes. Any suitable number of spray nozzles may be used for applying the liquid composition C onto the cellulose blank structure 2. The spray nozzles may be of any suitable construction for distributing the liquid composition C under hydraulic or pneumatic pressure. The arrangement of spray nozzles may differ from the ones described and illustrated, depending on the configuration, shape, and size of the cellulose blank structure 2. In the illustrated embodiment, the first surface S1 is an upper surface of the cellulose blank structure 2 and the second surface S2 is an opposite lower surface of the cellulose blank structure 2. In this embodiment, the liquid composition C is applied to both the upper surface and the lower surface of the cellulose blank structure 2. It should be understood that the liquid composition C applied to the first surface S1 and the second surface S2 may have the same chemical composition, or alternatively, a liquid composition C applied to the first surface S1 may have a chemical composition different from a liquid composition C applied to the second surface S2. Also in this embodiment, the liquid composition C could be applied onto cellulose blank structure 2 in two or more sub-steps in the same way as described above in connection to figures 3a-b.

The spray nozzles of the application unit 201 may spray the liquid composition C continuously or intermittently onto the cellulose blank structure 2. The liquid composition may also be applied over the whole cellulose blank structure 2 or only on parts or zones of the cellulose blank structure 2. The spray nozzles may suitably be arranged in a non-illustrated spray booth or similar structure, preventing that the liquid composition C when sprayed are spread into the surrounding environment.

In the embodiment illustrated in figure 5, the detection system 100 comprises two detection units 101 arranged on each side of the cellulose blank structure 2. Each detection unit 101 is arranged in connection to a corresponding detection area A_{D} of the product forming unit U, and the detection units 101 are used for detecting if the liquid composition C has been applied correctly on both sides of the cellulose blank structure. Each detection area A_{D} may have the same configurations as described in any of the different embodiments above, with the same control actions CA.

It should be understood that the cellulose product forming unit U may be configured in other ways. As an example, two or more detection units 101 or rows of detection units 101 may be used for detecting the liquid composition C. If the liquid composition C is applied onto the cellulose blank structure 2 in two or more sub-steps, a detection unit 101 or row of detection units 101 may be arranged after each sub-step or after a specific number of sub-steps. The detection results R_{D} from the detection units 101 are distributed to the control unit 102 for further analysis to form the analysis result R_{A}. The analysis result R_{A} may be arranged as a plurality of temperature images I that are compared to a plurality of reference temperature images I_{REF}.

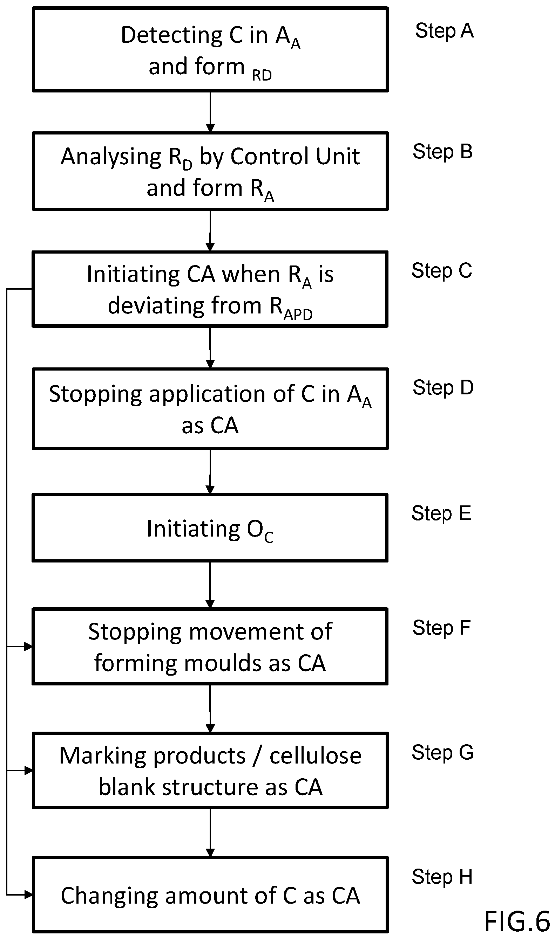

A flowchart of an exemplified method for detecting a liquid composition C applied onto a cellulose blank structure 2 is shown in figure 6. The exemplified method comprises the steps described below with reference to figure 6.

In step A, the applied liquid composition C is detected in the detection area A_{D} with the detection unit 101, and the detection by the detection unit 101 is forming a detection result R_{D}.

In step B, the detection result R_{D} is analysed by the control unit 102, and the analysis of the detection result R_{D} is forming an analysis result R_{A}.

In step C, a control action CA is initiated by the control unit 102 upon detection of an analysis result R_{A} deviating from a pre-determined analysis result R_{APD}.

In step D, the application of the liquid composition C onto the cellulose blank structure 2 by the application unit 201 in the application area A_{A} is stopped as the control action CA.

In step E, a cleaning operation O_{C} of the application unit 201 is initiated after stopping the application of the liquid composition C onto the cellulose blank structure 2 by the application unit 201.

In step F, a movement of the one or more forming moulds 3 is stopped as the control action CA.

In step G, one or more cellulose products 1 and/or the cellulose blank structure 2 are marked as the control action CA.

In step H, the amount of liquid composition C applied onto the cellulose blank structure in the application area A_{A} is changed as the control action CA.

According to the method, one or more control actions may be initiated upon detection of an analysis result R_{A} deviating from a pre-determined analysis result R_{APD}.

The present disclosure has been presented above with reference to specific embodiments. However, other embodiments than the above described are possible and within the scope of the disclosure. Different method steps than those described above, performing the method by hardware or software, may be provided within the scope of the disclosure. Thus, according to an exemplary embodiment, there is provided a non-transitory computer-readable storage medium storing one or more programs configured to be executed by one or more processors of the control unit, the one or more programs comprising instructions for performing the method according to any one of the above-discussed embodiments. Alternatively, according to another exemplary embodiment a cloud computing system can be configured to perform any of the method aspects presented herein. The cloud computing system may comprise distributed cloud computing resources that jointly perform the method aspects presented herein under control of one or more computer program products. Moreover, the processor may be connected to one or more communication interfaces and/or sensor interfaces for receiving and/transmitting data with external entities such as e.g. sensors, an off-site server, or a cloud-based server.

The processor or processors associated with the control may be or include any number of hardware components for conducting data or signal processing or for executing computer code stored in memory. The system may have an associated memory, and the memory may be one or more devices for storing data and/or computer code for completing or facilitating the various methods described in the present description. The memory may include volatile memory or non-volatile memory. The memory may include database components, object code components, script components, or any other type of information structure for supporting the various activities of the present description. According to an exemplary embodiment, any distributed or local memory device may be utilized with the systems and methods of this description. According to an exemplary embodiment the memory is communicably connected to the processor (e.g., via a circuit or any other wired, wireless, or network connection) and includes computer code for executing one or more processes described herein.

It will be appreciated that the above description is merely exemplary in nature and is not intended to limit the present disclosure, its application or uses. While specific examples have been described in the specification and illustrated in the drawings, it will be understood by those of ordinary skill in the art that various changes may be made without departing from the scope of the present disclosure as defined in the claims. Therefore, it is intended that the present disclosure not be limited to the particular examples illustrated by the drawings and described in the specification as the best mode presently contemplated for carrying out the teachings of the present disclosure, but that the scope of the present disclosure will include any embodiments falling within the scope of the appended claims. Reference signs mentioned in the claims should not be seen as limiting the extent of the matter protected by the claims, and their sole function is to make claims easier to understand.

### REFERENCE SIGNS

- 1:: Cellulose product
- 2:: Cellulose blank structure
- 3:: Forming mould
- 3a:: First mould part
- 3b:: Second mould part
- 100:: Detection system
- 101:: Detection unit
- 102:: Control unit
- 103:: Infrared detection sensor
- 200:: Application system
- 201:: Application unit

- A_{A}:: Application area
- A_{D}:: Detection area
- C:: Liquid composition
- CA:: Control action
- C_{DO}:: Desired operating condition
- C_{ND}:: Non-desired operating condition
- D_{F}:: Feeding direction
- I:: Temperature image
- I_{REF}:: Reference temperature image
- O_{C}:: Cleaning operation
- P:: Pressing module
- R_{D}:: Detection result
- R_{A}:: Analysis result
- R_{APD}:: Pre-determined analysis result
- S:: Surface
- T:: Temperature value
- T_{PD}:: Pre-defined temperature value
- TM:: Mean temperature value
- TM_{PD}:: Pre-defined mean temperature value
- U:: Cellulose product forming unit
- Z:: Temperature zone

## Claims

1. A method for detecting a liquid composition (C) applied onto a cellulose blank structure (2), with a detection system (100), wherein the detection system (100) is forming part of a cellulose product forming unit (U) for forming cellulose products (1) from the cellulose blank structure (2), wherein the detection system (100) comprises a thermal detection unit (101) connected to a control unit (102), wherein the method comprises the steps:
providing the cellulose blank structure (2) and feeding the cellulose blank structure (2) in a feeding direction (D_{F}), wherein the cellulose blank structure (2) upon feeding is moving through an application area (A_{A}) and a detection area (A_{D}), wherein the application area (A_{A}) is arranged upstream the detection area (A_{D});
applying the liquid composition (C) onto the cellulose blank structure (2) in the application area (A_{A}) in two or more sub-steps, wherein in each sub-step a part (C1, C2) of the liquid composition (C) is applied onto the cellulose blank structure (2), wherein a temperature (T1, T2) of each part (C1, C2) of the liquid composition (C) is different from the other parts (C1, C2) to determine if a temperature deviation in the cellulose blank structure (2) is dependent on a faulty application of either the first part (C1) or the second part (C2) of the liquid composition (C);
detecting the applied liquid composition (C) in the detection area (A_{D}) with the thermal detection unit (101), wherein the detection by the thermal detection unit (101) is forming a detection result (R_{D});
analysing the detection result (R_{D}) by the control unit (102), wherein the analysis of the detection result (R_{D}) is forming an analysis result (R_{A});
initiating a control action (CA) by the control unit (102) upon detection of an analysis result (R_{A}) deviating from a pre-determined analysis result (R_{APD}).

2. The method according to claim 1,
wherein the thermal detection unit (101) comprises an infrared detection sensor (103), wherein the method further comprises the steps: detecting the applied liquid composition (C) in the detection area (A_{D}) with the infrared detection sensor (103), wherein the detection result (R_{D}) is a temperature image (I) of the detection area; and analysing the temperature image (I) by the control unit (102) for forming the analysis result (R_{A}).

3. The method according to claim 2,
wherein the analysis result (R_{A}) comprises temperature values (T) of the detection area (A_{D}) and wherein the pre-determined analysis result (R_{APD}) comprises pre-defined temperature values (T_{PD}), wherein the method further comprises the step: initiating the control action (CA) by the control unit (102) when the analysis result (R_{A}) is a temperature value (T) in any part of the detection area (A_{D}) above or below the pre-defined temperature values (T_{PD}).

4. The method according to claim 2,
wherein the analysis result (R_{A}) comprises a mean temperature value (TM) of the detection area (A_{D}) and wherein the pre-determined analysis result (R_{APD}) is a pre-defined mean temperature value (TM_{PD}), wherein the method further comprises the step: initiating the control action (CA) by the control unit (102) when the analysis result (R_{A}) is a mean temperature value (TM) above or below the pre-defined mean temperature value (TM_{PD}).

5. The method according to any preceding claim,
wherein the method further comprises the step: continuously detecting the applied liquid composition (C) in the detection area (A_{D}) by the thermal detection unit (101).

6. The method according to any of claims 1 to 4,
wherein the method further comprises the step: intermittently detecting the applied liquid composition (C) in the detection area (A_{D}) by the thermal detection unit (101).

7. The method according to any preceding claim,
wherein the liquid composition (C) is applied onto the cellulose blank structure (2) in the application area (A_{A}) by an application unit (201), wherein the method further comprises the step: stopping the application of the liquid composition (C) onto the cellulose blank structure (2) by the application unit (201) in the application area (A_{A}) as the control action (CA).

8. The method according to claim 7,
wherein the method further comprises the step: initiating a cleaning operation (O_{C}) of the application unit (201) after stopping the application of the liquid composition (C) onto the cellulose blank structure (2) by the application unit (201).

9. The method according to any preceding claim,
wherein the cellulose product forming unit (U) comprises one or more forming moulds (3) for forming cellulose products (1), wherein the method further comprises the steps: stopping a movement of the one or more forming moulds (3) as the control action (CA).

10. The method according to any preceding claim,
wherein the method further comprises the steps: marking one or more cellulose products (1) and/or the cellulose blank structure (2) as the control action (CA).

11. The method according to any preceding claim,
wherein the method further comprises the steps: removing one or more cellulose products (1) and/or the cellulose blank structure (2) as the control action (CA).

12. The method according to any preceding claim,
wherein the method further comprises the steps: changing the amount of liquid composition (C) applied onto the cellulose blank structure (2) in the application area (A_{A}) as the control action (CA).

13. A detection system (100) for detecting a liquid composition (C) applied onto a cellulose blank structure (2) according to the method of any one of claims 1-12 upon feeding the cellulose blank structure (2) in a feeding direction (D_{F}) through an application area (A_{A}) and a detection area (A_{D}), wherein the detection system (100) is forming part of a cellulose product forming unit (U) for forming cellulose products (1) from the cellulose blank structure (2), wherein the detection system (100) comprises a thermal detection unit (101) connected to a control unit (102) to determine if a temperature deviation in the cellulose blank structure (2) is dependent on a faulty application of either a first part (C1) or a second part (C2) of the liquid composition (C),
wherein the thermal detection unit (101) is configured for detecting the applied liquid composition (C) in the detection area (A_{D}) arranged downstream an application area (A_{A}) in which the liquid composition (C) is applied onto the cellulose blank structure (2), wherein the detection by the thermal detection unit (101) is forming a detection result (R_{D});
wherein the control unit (102) is configured for analysing the detection result (R_{D}), wherein the analysis of the detection result (R_{D}) is forming an analysis result (R_{A}), and wherein the control unit (102) is configured for initiating a control action (CA) upon detection of an analysis result (R_{A}) deviating from a pre-determined analysis result (R_{APD}).

## Patentansprüche

1. Verfahren zum Detektieren einer auf eine Celluloserohlingsstruktur (2) aufgetragenen flüssigen Zusammensetzung (C) mit einem Detektionssystem (100), wobei das Detektionssystem (100) einen Teil einer Celluloseproduktbildungseinheit (U) zum Bilden von Celluloseprodukten (1) aus der Celluloserohlingsstruktur (2) bildet, wobei das Detektionssystem (100) eine Wärmedetektionseinheit (101) umfasst, die mit einer Steuereinheit (102) verbunden ist, wobei das Verfahren die folgenden Schritte umfasst:
Bereitstellen der Celluloserohlingsstruktur (2) und Einspeisen der Celluloserohlingsstruktur (2) in einer Einspeiserichtung (D_{F}), wobei sich die Celluloserohlingsstruktur (2) bei dem Einspeisen durch einen Auftragungsbereich (A_{A}) und einen Detektionsbereich (A_{D}) hindurch bewegt, wobei der Auftragungsbereich (A_{A}) dem Detektionsbereich (A_{D}) vorgelagert angeordnet ist;
Auftragen der flüssigen Zusammensetzung (C) auf die Celluloserohlingsstruktur (2) in dem Auftragungsbereich (A_{A}) in zwei oder mehreren Unterschritten, wobei in jedem Unterschritt ein Teil (C1, C2) der flüssigen Zusammensetzung (C) auf die Celluloserohlingsstruktur (2) aufgetragen wird, wobei sich eine Temperatur (T1, T2) jedes Teils (C1, C2) der flüssigen Zusammensetzung (C) von den anderen Teilen (C1, C2) unterscheidet, um zu bestimmen, ob eine Temperaturabweichung in der Celluloserohlingsstruktur (2) von einer fehlerhaften Auftragung entweder des ersten Teils (C1) oder des zweiten Teils (C2) der flüssigen Zusammensetzung (C) abhängt;
Detektieren der aufgetragenen flüssigen Zusammensetzung (C) in dem Detektionsbereich (A_{D}) mit der Wärmedetektionseinheit (101), wobei die Detektion durch die Wärmedetektionseinheit (101) ein Detektionsergebnis (R_{D}) bildet;
Analysieren des Detektionsergebnisses (R_{D}) durch die Steuereinheit (102), wobei die Analyse des Detektionsergebnisses (R_{D}) ein Analyseergebnis (R_{A}) bildet;
Initiieren einer Steuerhandlung (CA) durch die Steuereinheit (102) bei einer Detektion eines Analyseergebnisses (R_{A}), das von einem vorbestimmten Analyseergebnis (R_{APD}) abweicht.

2. Verfahren nach Anspruch 1,
wobei die Wärmedetektionseinheit (101) einen Infrarotdetektionssensor (103) umfasst, wobei das Verfahren ferner die folgenden Schritte umfasst: Detektieren der aufgetragenen flüssigen Zusammensetzung (C) in dem Detektionsbereich (A_{D}) mit dem Infrarotdetektionssensor (103), wobei das Detektionsergebnis (R_{D}) ein Temperaturbild (I) des Detektionsbereichs ist; und Analysieren des Temperaturbilds (I) durch die Steuereinheit (102) zum Bilden des Analyseergebnisses (R_{A}).

3. Verfahren nach Anspruch 2,
wobei das Analyseergebnis (R_{A}) Temperaturwerte (T) des Detektionsbereichs (A_{D}) umfasst und wobei das vorbestimmte Analyseergebnis (R_{APD}) vorbestimmte Temperaturwerte (T_{PD}) umfasst, wobei das Verfahren ferner den folgenden Schritt umfasst: Initiieren der Steuerhandlung (CA) durch die Steuereinheit (102), wenn das Analyseergebnis (R_{A}) ein Temperaturwert (T) in einem beliebigen Teil des Detektionsbereichs (A_{D}) über oder unter den vorbestimmten Temperaturwerten (T_{PD}) ist.

4. Verfahren nach Anspruch 2,
wobei das Analyseergebnis (R_{A}) einen mittleren Temperaturwert (TM) des Detektionsbereichs (A_{D}) umfasst und wobei das vorbestimmte Analyseergebnis (R_{APD}) ein vorbestimmter mittlerer Temperaturwert (TM_{PD}) ist, wobei das Verfahren ferner den folgenden Schritt umfasst: Initiieren der Steuerhandlung (CA) durch die Steuereinheit (102), wenn das Analyseergebnis (R_{A}) ein mittlerer Temperaturwert (TM) über oder unter dem vorbestimmten mittleren Temperaturwert (TM_{PD}) ist.

5. Verfahren nach einem der vorhergehenden Ansprüche,
wobei das Verfahren ferner den folgenden Schritt umfasst: kontinuierliches Detektieren der aufgetragenen flüssigen Zusammensetzung (C) in dem Detektionsbereich (A_{D}) durch die Wärmedetektionseinheit (101).

6. Verfahren nach einem der Ansprüche 1 bis 4,
wobei das Verfahren ferner den folgenden Schritt umfasst: intermittierendes Detektieren der aufgetragenen flüssigen Zusammensetzung (C) in dem Detektionsbereich (A_{D}) durch die Wärmedetektionseinheit (101).

7. Verfahren nach einem der vorhergehenden Ansprüche,
wobei die flüssige Zusammensetzung (C) durch eine Auftragungseinheit (201) auf die Celluloserohlingsstruktur (2) in dem Auftragungsbereich (A_{A}) aufgetragen wird, wobei das Verfahren ferner den folgenden Schritt umfasst: Stoppen der Auftragung der flüssigen Zusammensetzung (C) durch die Auftragungseinheit (201) auf die Celluloserohlingsstruktur (2) in dem Auftragungsbereich (A_{A}) als die Steuerhandlung (CA).

8. Verfahren nach Anspruch 7,
wobei das Verfahren ferner den folgenden Schritt umfasst: Initiieren eines Reinigungsvorgangs (O_{C}) der Auftragungseinheit (201) nach dem Stoppen der Auftragung der flüssigen Zusammensetzung (C) durch die Auftragungseinheit (201) auf die Celluloserohlingsstruktur (2).

9. Verfahren nach einem der vorhergehenden Ansprüche,
wobei die Celluloseproduktbildungseinheit (U) eine oder mehrere Bildungsformen (3) zum Bilden von Celluloseprodukten (1) umfasst, wobei das Verfahren ferner die folgenden Schritte umfasst: Stoppen einer Bewegung der einen oder der mehreren Bildungsformen (3) als die Steuerhandlung (CA).

10. Verfahren nach einem der vorhergehenden Ansprüche,
wobei das Verfahren ferner die folgenden Schritte umfasst: Markieren eines oder mehrerer Celluloseprodukte (1) und/oder der Celluloserohlingsstruktur (2) als die Steuerhandlung (CA).

11. Verfahren nach einem der vorhergehenden Ansprüche,
wobei das Verfahren ferner die folgenden Schritte umfasst: Entfernen eines oder mehrerer Celluloseprodukte (1) und/oder der Celluloserohlingsstruktur (2) als die Steuerhandlung (CA).

12. Verfahren nach einem der vorhergehenden Ansprüche,
wobei das Verfahren ferner die folgenden Schritte umfasst: Ändern der Menge der auf die Celluloserohlingsstruktur (2) aufgetragenen flüssigen Zusammensetzung (C) in dem Auftragungsbereich (A_{A}) als die Steuerhandlung (CA).

13. Detektionssystem (100) zum Detektieren einer auf eine Celluloserohlingsstruktur (2) aufgetragenen flüssigen Zusammensetzung (C) gemäß dem Verfahren nach einem der Ansprüche 1-12 bei dem Einspeisen der Celluloserohlingsstruktur (2) in einer Einspeiserichtung (D_{F}) durch einen Auftragungsbereich (A_{A}) und einen Detektionsbereich (A_{D}) hindurch, wobei das Detektionssystem (100) einen Teil einer Celluloseproduktbildungseinheit (U) zum Bilden von Celluloseprodukten (1) aus der Celluloserohlingsstruktur (2) bildet, wobei das Detektionssystem (100) eine Wärmedetektionseinheit (101) umfasst, die mit einer Steuereinheit (102) verbunden ist, um zu bestimmen, ob eine Temperaturabweichung in der Celluloserohlingsstruktur (2) von einer fehlerhaften Auftragung entweder eines ersten Teils (C1) oder eines zweiten Teils (C2) der flüssigen Zusammensetzung (C) abhängt,
wobei die Wärmedetektionseinheit (101) zum Detektieren der aufgetragenen flüssigen Zusammensetzung (C) in dem Detektionsbereich (A_{D}) konfiguriert ist, der einem Auftragungsbereich (A_{A}) nachgelagert angeordnet ist, in dem die flüssige Zusammensetzung (C) auf die Celluloserohlingsstruktur (2) aufgetragen wird, wobei die Detektion durch die Wärmedetektionseinheit (101) ein Detektionsergebnis (R_{D}) bildet;
wobei die Steuereinheit (102) zum Analysieren des Detektionsergebnisses (R_{D}) konfiguriert ist, wobei die Analyse des Detektionsergebnisses (R_{D}) ein Analyseergebnis (R_{A}) bildet und wobei die Steuereinheit (102) zum Initiieren einer Steuerhandlung (CA) bei einer Detektion eines Analyseergebnisses (R_{A}) konfiguriert ist, das von einem vorbestimmten Analyseergebnis (R_{APD}) abweicht.

## Revendications

1. Procédé de détection d'une composition liquide (C) appliquée sur une structure d'ébauche de cellulose (2) à l'aide d'un système de détection (100), dans lequel le système de détection (100) fait partie d'une unité de formage de produits en cellulose (U) pour former des produits en cellulose (1) à partir de la structure d'ébauche de cellulose (2), dans lequel le système de détection (100) comprend une unité de détection thermique (101) connectée à une unité de commande (102), dans lequel le procédé comprend les étapes consistant à :
fournir la structure d'ébauche de cellulose (2) et acheminer la structure d'ébauche de cellulose (2) dans une direction d'acheminement (D_{F}), dans lequel la structure d'ébauche de cellulose (2), lors de l'acheminement, se déplace à travers une zone d'application (A_{A}) et une zone de détection (A_{D}), dans lequel la zone d'application (A_{A}) est agencée en amont de la zone de détection (A_{D}) ;
appliquer la composition liquide (C) sur la structure d'ébauche de cellulose (2) dans la zone d'application (A_{A}) en au moins deux sous-étapes, dans lequel, dans chaque sous-étape, une partie (C1, C2) de la composition liquide (C) est appliquée sur la structure d'ébauche de cellulose (2), dans lequel une température (T1, T2) de chaque partie (C1, C2) de la composition liquide (C) est différente de celle des autres parties (C1, C2) pour déterminer si un écart de température dans la structure d'ébauche de cellulose (2) dépend d'une application défectueuse soit de la première partie (C1) soit de la seconde partie (C2) de la composition liquide (C) ;
détecter la composition liquide (C) appliquée dans la zone de détection (A_{D}) à l'aide de l'unité de détection thermique (101), dans lequel la détection par l'unité de détection thermique (101) génère un résultat de détection (R_{D}) ;
analyser le résultat de détection (R_{D}) par l'unité de commande (102), dans lequel l'analyse du résultat de détection (R_{D}) génère un résultat d'analyse (R_{A}) ;
déclencher une action de commande (CA) par l'unité de commande (102) lors de la détection d'un résultat d'analyse (R_{A}) s'écartant d'un résultat d'analyse prédéterminé (R_{APD}).

2. Procédé selon la revendication 1,
dans lequel l'unité de détection thermique (101) comprend un capteur de détection infrarouge (103), dans lequel le procédé comprend en outre les étapes consistant à : détecter la composition liquide (C) appliquée dans la zone de détection (A_{D}) avec le capteur de détection infrarouge (103), dans lequel le résultat de détection (R_{D}) est une image de température (I) de la zone de détection ; et analyser l'image de température (I) par l'unité de commande (102) pour générer le résultat d'analyse (R_{A}).

3. Procédé selon la revendication 2,
dans lequel le résultat d'analyse (R_{A}) comprend des valeurs de température (T) de la zone de détection (A_{D}) et dans lequel le résultat d'analyse prédéterminé (R_{APD}) comprend des valeurs de température prédéfinies (T_{PD}), dans lequel le procédé comprend en outre l'étape consistant à : déclencher l'action de commande (CA) par l'unité de commande (102) lorsque le résultat d'analyse (R_{A}) est une valeur de température (T) dans une partie quelconque de la zone de détection (A_{D}) supérieure ou inférieure aux valeurs de température prédéfinies (T_{PD}).

4. Procédé selon la revendication 2,
dans lequel le résultat d'analyse (R_{A}) comprend une valeur de température moyenne (TM) de la zone de détection (A_{D}) et dans lequel le résultat d'analyse prédéterminé (R_{APD}) est une valeur de température moyenne prédéfinie (TM_{PD}), dans lequel le procédé comprend en outre l'étape consistant à : déclencher l'action de commande (CA) par l'unité de commande (102) lorsque le résultat d'analyse (R_{A}) est une valeur de température moyenne (TM) supérieure ou inférieure à la valeur de température moyenne prédéfinie (TM_{PD}).

5. Procédé selon l'une quelconque des revendications précédentes,
dans lequel le procédé comprend en outre l'étape consistant à : détecter en continu la composition liquide (C) appliquée dans la zone de détection (A_{D}) par l'unité de détection thermique (101).

6. Procédé selon l'une quelconque des revendications 1 à 4, dans lequel le procédé comprend en outre l'étape consistant à : détecter par intermittence la composition liquide (C) appliquée dans la zone de détection (A_{D}) par l'unité de détection thermique (101).

7. Procédé selon l'une quelconque des revendications précédentes,
dans lequel la composition liquide (C) est appliquée sur la structure d'ébauche de cellulose (2) dans la zone d'application (A_{A}) par une unité d'application (201), dans lequel le procédé comprend en outre l'étape consistant à : arrêter l'application de la composition liquide (C) sur la structure d'ébauche de cellulose (2) par l'unité d'application (201) dans la zone d'application (A_{A}) en tant qu'action de commande (CA).

8. Procédé selon la revendication 7,
dans lequel le procédé comprend en outre l'étape consistant à : déclencher une opération de nettoyage (O_{c}) de l'unité d'application (201) après l'arrêt de l'application de la composition liquide (C) sur la structure d'ébauche de cellulose (2) par l'unité d'application (201).

9. Procédé selon l'une quelconque des revendications précédentes,
dans lequel l'unité de formage de produits en cellulose (U) comprend au moins un moule de formage (3) pour former des produits en cellulose (1), dans lequel le procédé comprend en outre l'étape consistant à : arrêter un mouvement dudit au moins un moule de formage (3) en tant qu'action de commande (CA).

10. Procédé selon l'une quelconque des revendications précédentes,
dans lequel le procédé comprend en outre l'étape consistant à : marquer au moins un produit en cellulose (1) et/ou la structure d'ébauche de cellulose (2) en tant qu'action de commande (CA).

11. Procédé selon l'une quelconque des revendications précédentes,
dans lequel le procédé comprend en outre l'étape consistant à : retirer au moins un produit en cellulose (1) et/ou la structure d'ébauche de cellulose (2) en tant qu'action de commande (CA).

12. Procédé selon l'une quelconque des revendications précédentes,
dans lequel le procédé comprend en outre l'étape consistant à : modifier la quantité de composition liquide (C) appliquée sur la structure d'ébauche de cellulose (2) dans la zone d'application (A_{A}) en tant qu'action de commande (CA).

13. Système de détection (100) pour détecter une composition liquide (C) appliquée sur une structure d'ébauche de cellulose (2) selon le procédé de l'une quelconque des revendications 1 à 12 lors de l'acheminement de la structure d'ébauche de cellulose (2) dans une direction d'acheminement (D_{F}) à travers une zone d'application (A_{A}) et une zone de détection (A_{D}), dans lequel le système de détection (100) fait partie d'une unité de formage de produits en cellulose (U) pour former des produits en cellulose (1) à partir de la structure d'ébauche de cellulose (2), dans lequel le système de détection (100) comprend une unité de détection thermique (101) connectée à une unité de commande (102) pour déterminer si un écart de température dans la structure d'ébauche de cellulose (2) dépend d'une application défectueuse soit d'une première partie (C1) soit d'une seconde partie (C2) de la composition liquide (C),
dans lequel l'unité de détection thermique (101) est configurée pour détecter la composition liquide (C) appliquée dans la zone de détection (A_{D}) agencée en aval d'une zone d'application (A_{A}) dans laquelle la composition liquide (C) est appliquée sur la structure d'ébauche de cellulose (2), dans lequel la détection par l'unité de détection thermique (101) génère un résultat de détection (R_{D}) ;
dans lequel l'unité de commande (102) est configurée pour analyser le résultat de détection (R_{D}), dans lequel l'analyse du résultat de détection (R_{D}) génère un résultat d'analyse (R_{A}), et dans lequel l'unité de commande (102) est configurée pour déclencher une action de commande (CA) suite à la détection d'un résultat d'analyse (R_{A}) s'écartant d'un résultat d'analyse prédéterminé (R_{APD}).
